(19) Europäisches Patentamt / European Patent Office / Office européen des brevets

(11) **EP 2 316 476 B1**

(12) **EUROPEAN PATENT SPECIFICATION**

(45) Date of publication and mention of the grant of the patent:
**25.11.2015 Bulletin 2015/48**

(51) Int Cl.:
**A61K 38/45** (2006.01) **A61K 38/19** (2006.01)
**A61K 38/18** (2006.01) **A61K 39/00** (2006.01)
**A61P 35/00** (2006.01)

(21) Application number: **10183808.4**

(22) Date of filing: **30.06.1999**

(54) **Antigenic peptides derived from telomerase**

Von der Telomerase abgeleitete antigene Peptide

Peptides antigènes dérivés de la télomérase

(84) Designated Contracting States:
**AT BE CH CY DE DK ES FI FR GB GR IE IT LI LU MC NL PT SE**

(30) Priority: **08.07.1998 NO 983141**

(43) Date of publication of application:
**04.05.2011 Bulletin 2011/18**

(62) Document number(s) of the earlier application(s) in accordance with Art. 76 EPC:
**10154215.7 / 2 258 383**
**03075681.1 / 1 362 597**
**99928238.7 / 1 093 381**

(73) Proprietor: **GemVax AS**
**0273 Oslo (NO)**

(72) Inventors:
• **Møller, Mona**
  **3925 Porsgrunn (NO)**
• **Eriksen, Jon Amund**
  **3916 Porsgrunn (NO)**
• **Gaudernack, Gustav**
  **0310 Oslo (NO)**
• **Gjertsen, Marianne Klemp**
  **1367 Snarøya (NO)**
• **Sæterdal, Ingvil**
  **1383 Asker (NO)**
• **Sæbøe-Larsen, Stein**
  **1165 Oslo (NO)**

(74) Representative: **Inspicos A/S**
**Kogle Allé 2**
**P.O. Box 45**
**2970 Hørsholm (DK)**

(56) References cited:
WO-A-98/14593    WO-A-98/21343
WO-A1-97/35619    WO-A1-98/01542
GB-A- 2 317 891

• **VONDERHEIDE R H ET AL: "THE TELOMERASE CATALYTIC SUBUNIT IS A WIDELY EXPRESSED TUMOR- ASSOCIATED ANTIGEN RECOGNIZED BY CYTOTOXIC T LYMPHOCYTES", IMMUNITY, CELL PRESS, US, vol. 10, no. 6, 1 June 1999 (1999-06-01), pages 673-679, XP000890114, ISSN: 1074-7613**
• **NAIR S K ET AL: "Induction of cytotoxic T cell responses and tumor immunity against unrelated tumors using telomerase reverse transcriptase RNA transfected dendritic cells.", NATURE MEDICINE SEP 2000 LNKD- PUBMED:10973321, vol. 6, no. 9, September 2000 (2000-09), pages 1011-1017, XP002600107, ISSN: 1078-8956**

SEQUENCE LISTING

<110> GemVax AS

<120> Antigenic Peptides Derived from Telomerase

<130> 16884EP02

<140> EP10154215.7
<141> 2010-02-22

<160> 254

<170> Patent In version 3.5

<210> 1
<211> 22
<212> PRT
<213> Homo sapiens

<400> 1

```
Phe Leu His Trp Leu Met Ser Val Tyr Val Val Glu Leu Leu Arg Ser
1               5                   10                  15

Phe Phe Tyr Val Thr Glu
            20
```

<210> 2
<211> 16
<212> PRT
<213> Homo sapiens

<400> 2

```
Glu Ala Arg Pro Ala Leu Leu Thr Ser Arg Leu Arg Phe Ile Pro Lys
1               5                   10                  15
```

<210> 3
<211> 16
<212> PRT
<213> Homo sapiens

<400> 3

```
Asp Gly Leu Arg Pro Ile Val Asn Met Asp Tyr Val Val Gly Ala Arg
1               5                   10                  15
```

<210> 4
<211> 18
<212> PRT
<213> Homo sapiens

<400> 4

```
Gly Val Pro Glu Tyr Gly Cys Val Val Asn Leu Arg Lys Thr Val Val
1               5                   10                  15

Asn Phe
```

<210> 5
<211> 23
<212> PRT
<213> Homo sapiens

<400> 5

```
Lys Phe Leu His Trp Leu Met Ser Val Tyr Val Val Glu Leu Leu Arg
1               5                   10                  15

Ser Phe Phe Tyr Val Thr Glu
                20
```

<210> 6
<211> 17
<212> PRT
<213> Homo sapiens

<400> 6

```
Lys Phe Leu His Trp Leu Met Ser Val Tyr Val Val Glu Leu Leu Arg
1               5                   10                  15

Ser
```

<210> 7
<211> 18
<212> PRT
<213> Homo sapiens

<400> 7

```
Leu Met Ser Val Tyr Val Val Glu Leu Leu Arg Ser Phe Phe Tyr Val
1               5                   10                  15

Thr Glu
```

<210> 8
<211> 9
<212> PRT
<213> Homo sapiens

<400> 8

```
Arg Glu Glu Ile Leu Ala Lys Phe Leu
1               5
```

<210> 9
<211> 9
<212> PRT
<213> Homo sapiens

<400> 9

```
Ile Leu Ala Lys Phe Leu His Trp Leu
1               5
```

<210> 10
<211> 9
<212> PRT
<213> Homo sapiens

<400> 10

```
Glu Leu Leu Arg Ser Phe Phe Tyr Val
1               5
```

<210> 11
<211> 9
<212> PRT
<213> Homo sapiens

<400> 11

```
Leu Met Ser Val Tyr Val Val Glu Leu
1               5
```

<210> 12
<211> 9
<212> PRT
<213> Homo sapiens

<400> 12

```
Thr Ser Arg Leu Arg Phe Ile Pro Lys
1               5
```

<210> 13
<211> 9
<212> PRT
<213> Homo sapiens

<400> 13

```
Leu Thr Ser Arg Leu Arg Phe Ile Pro
1               5
```

<210> 14
<211> 9
<212> PRT
<213> Homo sapiens

<400> 14

```
Leu Leu Thr Ser Arg Leu Arg Phe Ile
1               5
```

<210> 15
<211> 9
<212> PRT
<213> Homo sapiens

<400> 15

```
Ala Leu Leu Thr Ser Arg Leu Arg Phe
1               5
```

<210> 16
<211> 9
<212> PRT
<213> Homo sapiens

<400> 16

```
Pro Ala Leu Leu Thr Ser Arg Leu Arg
1               5
```

<210> 17
<211> 9
<212> PRT
<213> Homo sapiens

<400> 17

```
Arg Pro Ala Leu Leu Thr Ser Arg Leu
1               5
```

<210> 18
<211> 9
<212> PRT
<213> Homo sapiens

<400> 18

```
Ala Arg Pro Ala Leu Leu Thr Ser Arg
1               5
```

<210> 19
<211> 9
<212> PRT

<213> Homo sapiens

<400> 19

```
                          Glu Ala Arg Pro Ala Leu Leu Thr Ser
                          1               5
```

<210> 20
<211> 10
<212> PRT
<213> Homo sapiens

<400> 20

```
                          Leu Leu Arg Ser Phe Phe Tyr Val Thr Glu
                          1               5                   10
```

<210> 21
<211> 8
<212> PRT
<213> Homo sapiens

<400> 21

```
                          Ser Arg Leu Arg Phe Ile Pro Lys
                          1               5
```

<210> 22
<211> 12
<212> PRT
<213> Homo sapiens

<400> 22

```
                    Leu Arg Pro Ile Val Asn Met Asp Tyr Val Val Gly
                    1               5                   10
```

<210> 23
<211> 17
<212> PRT
<213> Homo sapiens

<400> 23

```
            Pro Glu Leu Tyr Phe Val Lys Val Asp Val Thr Gly Ala Tyr Asp Thr
            1               5                   10                  15

            Ile
```

<210> 24
<211> 24
<212> PRT
<213> Homo sapiens

<400> 24

```
Lys Ser Tyr Val Gln Cys Gln Gly Ile Pro Gln Gly Ser Ile Leu Ser
1               5               10              15

Thr Leu Leu Cys Ser Leu Cys Tyr
            20
```

<210> 25
<211> 13
<212> PRT
<213> Homo sapiens

<400> 25

```
Leu Leu Leu Arg Leu Val Asp Asp Phe Leu Leu Val Thr
1               5               10
```

<210> 26
<211> 11
<212> PRT
<213> Homo sapiens

<400> 26

```
Gly Cys Val Val Asn Leu Arg Lys Thr Val Val
1               5               10
```

<210> 27
<211> 48
<212> PRT
<213> Homo sapiens

<400> 27

```
Trp Leu Met Ser Val Tyr Val Val Glu Leu Leu Arg Ser Phe Phe Tyr
1               5               10              15

Val Thr Glu Thr Thr Phe Gln Lys Asn Arg Leu Phe Phe Tyr Arg Lys
            20              25              30

Ser Val Trp Ser Lys Leu Gln Ser Ile Gly Ile Arg Gln His Leu Lys
            35              40              45
```

<210> 28
<211> 25
<212> PRT
<213> Homo sapiens

<400> 28

```
Glu Val Arg Gln His Arg Glu Ala Arg Pro Ala Leu Leu Thr Ser Arg
1               5               10              15

Leu Arg Phe Ile Pro Lys Pro Asp Gly
            20              25
```

<210> 29
<211> 29
<212> PRT

<213> Homo sapiens

<400> 29

```
        Leu Arg Pro Ile Val Asn Met Asp Tyr Val Val Gly Ala Arg Thr Phe
        1               5                   10                  15

        Arg Arg Glu Lys Arg Ala Glu Arg Leu Thr Ser Arg Val
                    20                  25
```

<210> 30
<211> 34
<212> PRT
<213> Homo sapiens

<400> 30

```
        Pro Pro Pro Glu Leu Tyr Phe Val Lys Val Asp Val Thr Gly Ala Tyr
        1               5                   10                  15

        Asp Thr Ile Pro Gln Asp Arg Leu Thr Glu Val Ile Ala Ser Ile Ile
                    20                  25                  30

        Lys Pro
```

<210> 31
<211> 35
<212> PRT
<213> Homo sapiens

<400> 31

```
        Lys Ser Tyr Val Gln Cys Gln Gly Ile Pro Gln Gly Ser Ile Leu Ser
        1               5                   10                  15

        Thr Leu Leu Cys Ser Leu Cys Tyr Gly Asp Met Glu Asn Lys Leu Phe
                    20                  25                  30

        Ala Gly Ile
                    35
```

<210> 32
<211> 17
<212> PRT
<213> Homo sapiens

<400> 32

```
        Leu Leu Arg Leu Val Asp Asp Phe Leu Leu Val Thr Pro His Leu Thr
        1               5                   10                  15

        His
```

<210> 33
<211> 26
<212> PRT
<213> Homo sapiens

<400> 33

```
Ala Lys Thr Phe Leu Arg Thr Leu Val Arg Gly Val Pro Glu Tyr Gly
1               5                   10                  15

Cys Val Val Asn Leu Arg Lys Thr Val Val
            20                  25
```

<210> 34
<211> 11
<212> PRT
<213> Homo sapiens

<400> 34

```
His Gly Leu Phe Pro Trp Cys Gly Leu Leu Leu
1               5                   10
```

<210> 35
<211> 9
<212> PRT
<213> Homo sapiens

<400> 35

```
Tyr Val Val Glu Leu Leu Arg Ser Phe
1               5
```

<210> 36
<211> 9
<212> PRT
<213> Homo sapiens

<400> 36

```
Val Val Glu Leu Leu Arg Ser Phe Phe
1               5
```

<210> 37
<211> 9
<212> PRT
<213> Homo sapiens

<400> 37

```
Ser Val Tyr Val Val Glu Leu Leu Arg
1               5
```

<210> 38
<211> 9
<212> PRT
<213> Homo sapiens

<400> 38

```
Val Glu Leu Leu Arg Ser Phe Phe Tyr
1               5
```

<210> 39
<211> 9
<212> PRT
<213> Homo sapiens

<400> 39

```
Tyr Val Thr Glu Thr Thr Phe Gln Lys
1               5
```

<210> 40
<211> 9
<212> PRT
<213> Homo sapiens

<400> 40

```
Arg Leu Phe Phe Tyr Arg Lys Ser Val
1               5
```

<210> 41
<211> 9
<212> PRT
<213> Homo sapiens

<400> 41

```
Ser Ile Gly Ile Arg Gln His Leu Lys
1               5
```

<210> 42
<211> 9
<212> PRT
<213> Homo sapiens

<400> 42

```
Gly Val Pro Glu Tyr Gly Cys Val Val
1               5
```

<210> 43
<211> 9
<212> PRT
<213> Homo sapiens

<400> 43

```
Val Val Asn Leu Arg Lys Thr Val Val
1               5
```

<210> 44
<211> 9
<212> PRT
<213> Homo sapiens

<400> 44

Gly Leu Phe Pro Trp Cys Gly Leu Leu
1               5

<210> 45
<211> 9
<212> PRT
<213> Homo sapiens

<400> 45

Arg Pro Ile Val Asn Met Asp Tyr Val
1               5

<210> 46
<211> 9
<212> PRT
<213> Homo sapiens

<400> 46

Leu Arg Pro Ile Val Asn Met Asp Tyr
1               5

<210> 47
<211> 9
<212> PRT
<213> Homo sapiens

<400> 47

Tyr Val Val Gly Ala Arg Thr Phe Arg
1               5

<210> 48
<211> 9
<212> PRT
<213> Homo sapiens

<400> 48

Ala Arg Thr Phe Arg Arg Glu Lys Arg
1               5

<210> 49
<211> 9
<212> PRT
<213> Homo sapiens

<400> 49

Val Val Gly Ala Arg Thr Phe Arg Arg
1               5

<210> 50
<211> 9
<212> PRT
<213> Homo sapiens

<400> 50

Gly Ala Arg Thr Phe Arg Arg Glu Lys
1               5

<210> 51
<211> 9
<212> PRT
<213> Homo sapiens

<400> 51

Ala Arg Thr Phe Arg Arg Glu Lys Pro
1               5

<210> 52
<211> 9
<212> PRT
<213> Homo sapiens

<400> 52

Pro Pro Glu Leu Tyr Phe Val Lys Val
1               5

<210> 53
<211> 9
<212> PRT
<213> Homo sapiens

<400> 53

Glu Leu Tyr Phe Val Lys Val Asp Val
1               5

<210> 54
<211> 9
<212> PRT
<213> Homo sapiens

<400> 54

Phe Val Lys Val Asp Val Thr Gly Ala
1               5

<210> 55
<211> 9
<212> PRT
<213> Homo sapiens

<400> 55

```
                                        Ile Pro Gln Asp Arg Leu Thr Glu Val
                                        1               5
```

<210> 56
<211> 9
<212> PRT
<213> Homo sapiens

<400> 56

```
                                        Asp Arg Leu Thr Glu Val Ile Ala Ser
                                        1               5
```

<210> 57
<211> 9
<212> PRT
<213> Homo sapiens

<400> 57

```
                                        Arg Leu Thr Glu Val Ile Ala Ser Ile
                                        1               5
```

<210> 58
<211> 10
<212> PRT
<213> Homo sapiens

<400> 58

```
                                        Ile Pro Gln Gly Ser Ile Leu Ser Thr Leu
                                        1               5                   10
```

<210> 59
<211> 9
<212> PRT
<213> Homo sapiens

<400> 59

```
                                        Ile Leu Ser Thr Leu Leu Cys Ser Leu
                                        1               5
```

<210> 60
<211> 9
<212> PRT
<213> Homo sapiens

<400> 60

```
                                        Leu Leu Arg Leu Val Asp Asp Phe Leu
                                        1               5
```

<210> 61
<211> 9
<212> PRT
<213> Homo sapiens

```
                                        Ile Pro Gln Asp Arg Leu Thr Glu Val
                                        1               5
```

<400> 61

```
                            Arg Leu Val Asp Asp Phe Leu Leu Val
                            1               5
```

<210> 62
<211> 9
<212> PRT
<213> Homo sapiens

<400> 62

```
                            Val Pro Glu Tyr Gly Cys Val Val Asn
                            1               5
```

<210> 63
<211> 10
<212> PRT
<213> Homo sapiens

<400> 63

```
                        Val Pro Glu Tyr Gly Cys Val Val Asn Leu
                        1               5                   10
```

<210> 64
<211> 9
<212> PRT
<213> Homo sapiens

<400> 64

```
                            Thr Leu Val Arg Gly Val Pro Glu Tyr
                            1               5
```

<210> 65
<211> 9
<212> PRT
<213> Homo sapiens

<400> 65

```
                            Phe Leu Arg Thr Leu Val Arg Gly Val
                            1               5
```

<210> 66
<211> 9
<212> PRT
<213> Homo sapiens

<400> 66

```
                            Ala Glu Val Arg Gln His Arg Glu Ala
                            1               5
```

<210> 67
<211> 9
<212> PRT

<213> Homo sapiens

<400> 67

Arg Glu Ala Gly Val Pro Leu Gly Leu
1               5

<210> 68
<211> 9
<212> PRT
<213> Homo sapiens

<400> 68

Glu Glu Ala Thr Ser Leu Glu Gly Ala
1               5

<210> 69
<211> 9
<212> PRT
<213> Homo sapiens

<400> 69

Tyr Ala Glu Thr Lys His Phe Leu Tyr
1               5

<210> 70
<211> 9
<212> PRT
<213> Homo sapiens

<400> 70

Ile Ser Asp Thr Ala Ser Leu Cys Tyr
1               5

<210> 71
<211> 9
<212> PRT
<213> Homo sapiens

<400> 71

Asp Thr Asp Pro Arg Arg Leu Val Gln
1               5

<210> 72
<211> 9
<212> PRT
<213> Homo sapiens

<400> 72

Ala Gln Asp Pro Pro Pro Glu Leu Tyr
1               5

<210> 73
<211> 9
<212> PRT
<213> Homo sapiens

<400> 73

Leu Thr Asp Leu Gln Pro Tyr Met Arg
1               5

<210> 74
<211> 9
<212> PRT
<213> Homo sapiens

<400> 74

Gln Ser Asp Tyr Ser Ser Tyr Ala Arg
1               5

<210> 75
<211> 9
<212> PRT
<213> Homo sapiens

<400> 75

Leu Leu Ala Arg Cys Ala Leu Phe Val
1               5

<210> 76
<211> 9
<212> PRT
<213> Homo sapiens

<400> 76

Trp Leu Cys His Gln Ala Phe Leu Leu
1               5

<210> 77
<211> 9
<212> PRT
<213> Homo sapiens

<400> 77

Arg Leu Val Asp Asp Phe Leu Leu Val
1               5

<210> 78
<211> 9
<212> PRT
<213> Homo sapiens

<400> 78

Leu Glu Ala Ala Ala Asn Pro Ala Leu
1                   5

<210> 79
<211> 9
<212> PRT
<213> Homo sapiens

<400> 79

Leu Gln Leu Pro Phe His Gln Gln Val
1                   5

<210> 80
<211> 9
<212> PRT
<213> Homo sapiens

<400> 80

Arg Leu Gly Pro Gln Gly Trp Arg Leu
1                   5

<210> 81
<211> 9
<212> PRT
<213> Homo sapiens

<400> 81

Ser Leu Gln Glu Leu Thr Trp Lys Met
1                   5

<210> 82
<211> 9
<212> PRT
<213> Homo sapiens

<400> 82

Asn Val Leu Ala Phe Gly Phe Ala Leu
1                   5

<210> 83
<211> 9
<212> PRT
<213> Homo sapiens

<400> 83

Val Leu Leu Lys Thr His Cys Pro Leu
1                   5

<210> 84
<211> 9
<212> PRT

<213> Homo sapiens

<400> 84

Phe Leu Leu Val Thr Pro His Leu Thr
1               5

<210> 85
<211> 9
<212> PRT
<213> Homo sapiens

<400> 85

Thr Leu Thr Asp Leu Gln Pro Tyr Met
1               5

<210> 86
<211> 9
<212> PRT
<213> Homo sapiens

<400> 86

Gln Glu Thr Ser Pro Leu Arg Asp Ala
1               5

<210> 87
<211> 9
<212> PRT
<213> Homo sapiens

<400> 87

Phe Leu Asp Leu Gln Val Asn Ser Leu
1               5

<210> 88
<211> 9
<212> PRT
<213> Homo sapiens

<400> 88

Ser Leu Asn Glu Ala Ser Ser Gly Leu

1               5

<210> 89
<211> 9
<212> PRT
<213> Homo sapiens

<400> 89

Arg Glu Val Leu Pro Leu Ala Thr Phe
1 5

<210> 90
<211> 9
<212> PRT
<213> Homo sapiens

<400> 90

Leu Leu Gly Ala Ser Val Leu Gly Leu
1 5

<210> 91
<211> 9
<212> PRT
<213> Homo sapiens

<400> 91

Val Leu Ala Phe Gly Phe Ala Leu Leu
1 5

<210> 92
<211> 9
<212> PRT
<213> Homo sapiens

<400> 92

Leu Gln Pro Tyr Met Arg Gln Phe Val
1 5

<210> 93
<211> 9
<212> PRT
<213> Homo sapiens

<400> 93

Leu Met Ser Val Tyr Val Val Glu Leu
1 5

<210> 94
<211> 9
<212> PRT
<213> Homo sapiens

<400> 94

Arg Leu Pro Gln Arg Tyr Trp Gln Met
1 5

<210> 95
<211> 9
<212> PRT
<213> Homo sapiens

<400> 95

Arg Gln His Ser Ser Pro Trp Gln Val
1               5

<210> 96
<211> 9
<212> PRT
<213> Homo sapiens

<400> 96

Arg Gln His Ser Ser Pro Trp Gln Val
1               5

<210> 97
<211> 9
<212> PRT
<213> Homo sapiens

<400> 97

Tyr Leu Pro Asn Thr Val Thr Asp Ala
1               5

<210> 98
<211> 9
<212> PRT
<213> Homo sapiens

<400> 98

Asn Met Arg Arg Lys Leu Phe Gly Val
1               5

<210> 99
<211> 9
<212> PRT
<213> Homo sapiens

<400> 99

Arg Leu Thr Ser Arg Val Lys Ala Leu
1               5

<210> 100
<211> 9
<212> PRT
<213> Homo sapiens

<400> 100

Leu Leu Gln Ala Tyr Arg Phe His Ala
1               5

<210> 101
<211> 9

<212> PRT
<213> Homo sapiens

<400> 101

```
Leu Leu Asp Thr Arg Thr Leu Glu Val
1               5
```

<210> 102
<211> 9
<212> PRT
<213> Homo sapiens

<400> 102

```
Tyr Met Arg Gln Phe Val Ala His Leu
1               5
```

<210> 103
<211> 9
<212> PRT
<213> Homo sapiens

<400> 103

```
Lys Glu Gln Leu Arg Pro Ser Phe Leu
1               5
```

<210> 104
<211> 9
<212> PRT
<213> Homo sapiens

<400> 104

```
Cys Leu Val Cys Val Pro Trp Asp Ala
1               5
```

<210> 105
<211> 9
<212> PRT
<213> Homo sapiens

<400> 105

```
Leu Leu Ser Ser Leu Arg Pro Ser Leu
1               5
```

<210> 106
<211> 9
<212> PRT
<213> Homo sapiens

<400> 106

```
                              Phe Met Cys His His Ala Val Arg Ile
                              1               5
```

<210> 107
<211> 9
<212> PRT
<213> Homo sapiens

<400> 107

```
                              Leu Gln Val Asn Ser Leu Gln Thr Val
                              1               5
```

<210> 108
<211> 9
<212> PRT
<213> Homo sapiens

<400> 108

```
                              Leu Val Ala Gln Cys Leu Val Cys Val
                              1               5
```

<210> 109
<211> 9
<212> PRT
<213> Homo sapiens

<400> 109

```
                              Cys Leu Lys Glu Leu Val Ala Arg Val
                              1               5
```

<210> 110
<211> 9
<212> PRT
<213> Homo sapiens

<400> 110

```
                              Phe Leu Arg Asn Thr Lys Lys Phe Ile
                              1               5
```

<210> 111
<211> 9
<212> PRT
<213> Homo sapiens

<400> 111

```
                              Ala Leu Pro Ser Asp Phe Lys Thr Ile
                              1               5
```

<210> 112
<211> 9
<212> PRT
<213> Homo sapiens

<400> 112

Val Leu Val His Leu Leu Ala Arg Cys
1               5

<210> 113
<211> 9
<212> PRT
<213> Homo sapiens

<400> 113

Val Gln Ser Asp Tyr Ser Ser Tyr Ala
1               5

<210> 114
<211> 9
<212> PRT
<213> Homo sapiens

<400> 114

Ser Val Trp Ser Lys Leu Gln Ser Ile
1               5

<210> 115
<211> 9
<212> PRT
<213> Homo sapiens

<400> 115

Lys Leu Pro Gly Thr Thr Leu Thr Ala
1               5

<210> 116
<211> 9
<212> PRT
<213> Homo sapiens

<400> 116

Gln Leu Ser Arg Lys Leu Pro Gly Thr
1               5

<210> 117
<211> 9
<212> PRT
<213> Homo sapiens

<400> 117

Arg Glu Lys Pro Gln Gly Ser Val Ala
1               5

<210> 118
<211> 9
<212> PRT

<213> Homo sapiens

<400> 118

Gly Leu Leu Leu Asp Thr Arg Thr Leu
1                   5

<210> 119
<211> 9
<212> PRT
<213> Homo sapiens

<400> 119

Trp Met Pro Gly Thr Pro Arg Arg Leu
1                   5

<210> 120
<211> 9
<212> PRT
<213> Homo sapiens

<400> 120

Ser Leu Thr Gly Ala Arg Arg Leu Val
1                   5

<210> 121
<211> 9
<212> PRT
<213> Homo sapiens

<400> 121

Val Val Ile Glu Gln Ser Ser Ser Leu
1                   5

<210> 122
<211> 9
<212> PRT
<213> Homo sapiens

<400> 122

Leu Pro Ser Glu Ala Val Gln Trp Leu
1                   5

<210> 123
<211> 9
<212> PRT
<213> Homo sapiens

<400> 123

Gln Ala Tyr Arg Phe His Ala Cys Val
1                   5

<210> 124

<211> 9
<212> PRT
<213> Homo sapiens

<400> 124

```
Gly Leu Phe Asp Val Phe Leu Arg Phe
1               5
```

<210> 125
<211> 9
<212> PRT
<213> Homo sapiens

<400> 125

```
Lys Leu Phe Gly Val Leu Arg Leu Lys
1               5
```

<210> 126
<211> 9
<212> PRT
<213> Homo sapiens

<400> 126

```
Arg Leu Arg Glu Glu Ile Leu Ala Lys
1               5
```

<210> 127
<211> 9
<212> PRT
<213> Homo sapiens

<400> 127

```
Leu Glu Val Gln Ser Asp Tyr Ser Ser
1               5
```

<210> 128
<211> 9
<212> PRT
<213> Homo sapiens

<400> 128

```
Gly Leu Pro Ala Pro Gly Ala Arg Arg
1               5
```

<210> 129
<211> 9
<212> PRT
<213> Homo sapiens

<400> 129

```
Gly Leu Phe Pro Trp Cys Gly Leu Leu
1               5
```

<210> 130
<211> 9
<212> PRT
<213> Homo sapiens

<400> 130

```
Lys Leu Thr Arg His Arg Val Thr Tyr
1               5
```

<210> 131
<211> 9
<212> PRT
<213> Homo sapiens

<400> 131

```
Val Leu Pro Leu Ala Thr Phe Val Arg
1               5
```

<210> 132
<211> 9
<212> PRT
<213> Homo sapiens

<400> 132

```
Glu Leu Val Ala Arg Val Leu Gln Arg
1               5
```

<210> 133
<211> 9
<212> PRT
<213> Homo sapiens

<400> 133

```
Asp Pro Arg Arg Leu Val Gln Leu Leu
1               5
```

<210> 134
<211> 9
<212> PRT
<213> Homo sapiens

<400> 134

```
Phe Val Arg Ala Cys Leu Arg Arg Leu
1               5
```

<210> 135
<211> 9
<212> PRT

<213> Homo sapiens

<400> 135

```
                                        Ser Val Arg Glu Ala Gly Val Pro Leu
                                        1               5
```

<210> 136
<211> 9
<212> PRT
<213> Homo sapiens

<400> 136

```
                                        Ala Gly Arg Asn Met Arg Arg Lys Leu
                                        1               5
```

<210> 137
<211> 9
<212> PRT
<213> Homo sapiens

<400> 137

```
                                        Leu Ala Arg Cys Ala Leu Phe Val Leu
                                        1               5
```

<210> 138
<211> 9
<212> PRT
<213> Homo sapiens

<400> 138

```
                                        Arg Pro Ala Glu Glu Ala Thr Ser Leu
                                        1               5
```

<210> 139
<211> 9
<212> PRT
<213> Homo sapiens

<400> 139

```
                                        Leu Pro Ser Asp Phe Lys Thr Ile Leu
                                        1               5
```

<210> 140
<211> 9
<212> PRT
<213> Homo sapiens

<400> 140

```
                                        Leu Pro Ser Glu Ala Val Gln Trp Leu
                                        1               5
```

<210> 141
<211> 9
<212> PRT
<213> Homo sapiens

<400> 141

```
Leu Pro Gly Thr Thr Leu Thr Ala Leu
1               5
```

<210> 142
<211> 9
<212> PRT
<213> Homo sapiens

<400> 142

```
Arg Pro Ser Phe Leu Leu Ser Ser Leu
1               5
```

<210> 143
<211> 9
<212> PRT
<213> Homo sapiens

<400> 143

```
Leu Pro Asn Thr Val Thr Asp Ala Leu
1               5
```

<210> 144
<211> 9
<212> PRT
<213> Homo sapiens

<400> 144

```
Arg Glu Ala Arg Pro Ala Leu Leu Thr
1               5
```

<210> 145
<211> 9
<212> PRT
<213> Homo sapiens

<400> 145

```
Arg Cys Arg Ala Val Arg Ser Leu Leu
1               5
```

<210> 146
<211> 9
<212> PRT
<213> Homo sapiens

<400> 146

```
                    Met Pro Arg Ala Pro Arg Cys Arg Ala
                    1               5
```

<210> 147
<211> 9
<212> PRT
<213> Homo sapiens

<400> 147

```
                    Gly Ile Arg Arg Asp Gly Leu Leu Leu


                         1               5
```

<210> 148
<211> 9
<212> PRT
<213> Homo sapiens

<400> 148

```
                    Val Leu Arg Leu Lys Cys His Ser Leu
                    1               5
```

<210> 149
<211> 9
<212> PRT
<213> Homo sapiens

<400> 149

```
                    Tyr Met Arg Gln Phe Val Ala His Leu
                    1               5
```

<210> 150
<211> 9
<212> PRT
<213> Homo sapiens

<400> 150

```
                    Ser Leu Arg Thr Ala Gln Thr Gln Leu
                    1               5
```

<210> 151
<211> 9
<212> PRT
<213> Homo sapiens

<400> 151

```
                    Gln Met Arg Pro Leu Phe Leu Glu Leu
                    1               5
```

<210> 152
<211> 9

<212> PRT
<213> Homo sapiens

<400> 152

```
Glu Glu Asp Thr Asp Pro Arg Arg Leu
1               5
```

<210> 153
<211> 9
<212> PRT
<213> Homo sapiens

<400> 153

```
Phe Val Gln Met Pro Ala His Gly Leu
1               5
```

<210> 154
<211> 9
<212> PRT
<213> Homo sapiens

<400> 154

```
His Ala Ser Gly Pro Arg Arg Arg Leu
1               5
```

<210> 155
<211> 9
<212> PRT
<213> Homo sapiens

<400> 155

```
Val Val Ile Glu Gln Ser Ser Ser Leu
1               5
```

<210> 156
<211> 9
<212> PRT
<213> Homo sapiens

<400> 156

```
Arg Val Ile Ser Asp Thr Ala Ser Leu
1               5
```

<210> 157
<211> 9
<212> PRT
<213> Homo sapiens

<400> 157

```
Cys Val Pro Ala Ala Glu His Arg Leu
1               5
```

<210> 158
<211> 9
<212> PRT
<213> Homo sapiens

<400> 158

Arg Val Lys Ala Leu Phe Ser Val Leu
1               5

<210> 159
<211> 9
<212> PRT
<213> Homo sapiens

<400> 159

Asn Val Leu Ala Phe Gly Phe Ala Leu
1               5

<210> 160
<211> 9
<212> PRT
<213> Homo sapiens

<400> 160

Leu Val Ala Arg Val Leu Gln Arg Leu
1               5

<210> 161
<211> 9
<212> PRT
<213> Homo sapiens

<400> 161

Phe Ala Gly Ile Arg Arg Asp Gly Leu
1               5

<210> 162
<211> 9
<212> PRT
<213> Homo sapiens

<400> 162

His Ala Gln Cys Pro Tyr Gly Val Leu
1               5

<210> 163
<211> 9
<212> PRT
<213> Homo sapiens

<400> 163

```
Arg Ala Gln Asp Pro Pro Pro Glu Leu
1               5
```

<210> 164
<211> 9
<212> PRT
<213> Homo sapiens

<400> 164

```
Ala Tyr Arg Phe His Ala Cys Val Leu
1               5
```

<210> 165
<211> 9
<212> PRT
<213> Homo sapiens

<400> 165

```
His Ala Lys Leu Ser Leu Gln Glu Leu
1               5
```

<210> 166
<211> 9
<212> PRT
<213> Homo sapiens

<400> 166

```
Gly Ala Lys Gly Ala Ala Gly Pro Leu
1               5
```

<210> 167
<211> 9
<212> PRT
<213> Homo sapiens

<400> 167

```
Thr Ala Ser Leu Cys Tyr Ser Ile Leu
1               5
```

<210> 168
<211> 9
<212> PRT
<213> Homo sapiens

<400> 168

```
Ala Pro Arg Cys Arg Ala Val Arg Ser
1               5
```

<210> 169

<211> 9
<212> PRT
<213> Homo sapiens

<400> 169

```
Gly Ala Arg Arg Leu Val Glu Thr Ile
1               5
```

<210> 170
<211> 9
<212> PRT
<213> Homo sapiens

<400> 170

```
Ala Gln Cys Pro Tyr Gly Val Leu Leu
1               5
```

<210> 171
<211> 9
<212> PRT
<213> Homo sapiens

<400> 171

```
His Ala Lys Thr Phe Leu Arg Thr Leu
1               5
```

<210> 172
<211> 9
<212> PRT
<213> Homo sapiens

<400> 172

```
Glu Ala Thr Ser Leu Glu Gly Ala Leu
1               5
```

<210> 173
<211> 9
<212> PRT
<213> Homo sapiens

<400> 173

```
Lys Ala Lys Asn Ala Gly Met Ser Leu
1               5
```

<210> 174
<211> 9
<212> PRT
<213> Homo sapiens

<400> 174

```
Ala Gln Thr Gln Leu Ser Arg Lys Leu
1               5
```

<210> 175
<211> 9
<212> PRT
<213> Homo sapiens

<400> 175

```
Ala Gly Ile Arg Arg Asp Gly Leu Leu
1                   5
```

<210> 176
<211> 9
<212> PRT
<213> Homo sapiens

<400> 176

```
Val Leu Arg Leu Lys Cys His Ser Leu
1                   5
```

<210> 177
<211> 9
<212> PRT
<213> Homo sapiens

<400> 177

```
Ile Leu Lys Ala Lys Asn Ala Gly Met
1                   5
```

<210> 178
<211> 9
<212> PRT
<213> Homo sapiens

<400> 178

```
Asp Pro Arg Arg Leu Val Gln Leu Leu
1                   5
```

<210> 179
<211> 9
<212> PRT
<213> Homo sapiens

<400> 179

```
Gly Ala Lys Gly Ala Ala Gly Pro Leu
1                   5
```

<210> 180
<211> 9
<212> PRT
<213> Homo sapiens

<400> 180

```
                         Phe Ala Gly Ile Arg Arg Asp Gly Leu
                         1               5
```

<210> 181
<211> 9
<212> PRT
<213> Homo sapiens

<400> 181

```
                         Gly Ala Arg Arg Arg Gly Gly Ser Ala
                         1               5
```

<210> 182
<211> 9
<212> PRT
<213> Homo sapiens

<400> 182

```
                         His Ala Lys Thr Phe Leu Arg Thr Leu
                         1               5
```

<210> 183
<211> 9
<212> PRT
<213> Homo sapiens

<400> 183

```
                         His Ala Lys Leu Ser Leu Gln Glu Leu
                         1               5
```

<210> 184
<211> 9
<212> PRT
<213> Homo sapiens

<400> 184

```
                         Leu Ala Arg Cys Ala Leu Phe Val Leu
                         1               5
```

<210> 185
<211> 9
<212> PRT
<213> Homo sapiens

<400> 185

```
                         Glu His Arg Leu Arg Glu Glu Ile Leu
                         1               5
```

<210> 186
<211> 9
<212> PRT
<213> Homo sapiens

<400> 186

```
Asn Met Arg Arg Lys Leu Phe Gly Val
1               5
```

<210> 187
<211> 9
<212> PRT
<213> Homo sapiens

<400> 187

```
Cys Ala Arg Glu Lys Pro Gln Gly Ser
1               5
```

<210> 188
<211> 9
<212> PRT
<213> Homo sapiens

<400> 188

```
Leu Thr Arg His Arg Val Thr Tyr Val
1               5
```

<210> 189
<211> 9
<212> PRT
<213> Homo sapiens

<400> 189

```
Arg Arg Phe Leu Arg Asn Thr Lys Lys
1               5
```

<210> 190
<211> 9
<212> PRT
<213> Homo sapiens

<400> 190

```
Arg Arg Asp Gly Leu Leu Leu Arg Leu
1               5
```

<210> 191
<211> 9
<212> PRT
<213> Homo sapiens

<400> 191

```
Arg Arg Glu Lys Arg Ala Glu Arg Leu
1               5
```

<210> 192
<211> 9
<212> PRT
<213> Homo sapiens

<400> 192

```
Arg Arg Leu Val Glu Thr Ile Phe Leu
1               5
```

<210> 193
<211> 9
<212> PRT
<213> Homo sapiens

<400> 193

```
Leu Arg Phe Met Cys His His Ala Val
1               5
```

<210> 194
<211> 9
<212> PRT
<213> Homo sapiens

<400> 194

```
Arg Arg Tyr Ala Val Val Gln Lys Ala
1               5
```

<210> 195
<211> 9
<212> PRT
<213> Homo sapiens

<400> 195

```
Lys Arg Ala Glu Arg Leu Thr Ser Arg
1               5
```

<210> 196
<211> 9
<212> PRT
<213> Homo sapiens

<400> 196

```
Arg Arg Lys Leu Phe Gly Val Leu Arg
1               5
```

<210> 197
<211> 9
<212> PRT
<213> Homo sapiens

<400> 197

```
                        Arg Arg Arg Gly Gly Ser Ala Ser Arg
                        1               5
```

<210> 198
<211> 9
<212> PRT
<213> Homo sapiens

<400> 198

```
                        Arg Arg Leu Pro Arg Leu Pro Gln Arg
                        1               5
```

<210> 199
<211> 9
<212> PRT
<213> Homo sapiens

<400> 199

```
                        Arg Arg Leu Gly Pro Gln Gly Trp Arg
                        1               5
```

<210> 200
<211> 9
<212> PRT
<213> Homo sapiens

<400> 200

```
                        Leu Arg Gly Ser Gly Ala Trp Gly Leu
                        1               5
```

<210> 201
<211> 9
<212> PRT
<213> Homo sapiens

<400> 201

```
                        His Arg Glu Ala Arg Pro Ala Leu Leu
                        1               5
```

<210> 202
<211> 9
<212> PRT
<213> Homo sapiens

<400> 202

```
                        Val Arg Arg Tyr Ala Val Val Gln Lys
                        1               5
```

<210> 203
<211> 9
<212> PRT

<213> Homo sapiens

<400> 203

Ala Arg Thr Ser Ile Arg Ala Ser Leu
1               5

<210> 204
<211> 9
<212> PRT
<213> Homo sapiens

<400> 204

His Arg Val Thr Tyr Val Pro Leu Leu
1               5

<210> 205
<211> 9
<212> PRT
<213> Homo sapiens

<400> 205

Leu Arg Ser His Tyr Arg Glu Val Leu
1               5

<210> 206
<211> 9
<212> PRT
<213> Homo sapiens

<400> 206

Met Arg Pro Leu Phe Leu Glu Leu Leu

1               5

<210> 207
<211> 9
<212> PRT
<213> Homo sapiens

<400> 207

His Arg Ala Trp Arg Thr Phe Val Leu
1               5

<210> 208
<211> 9
<212> PRT
<213> Homo sapiens

<400> 208

```
Met Arg Arg Lys Leu Phe Gly Val Leu
1               5
```

<210> 209
<211> 9
<212> PRT
<213> Homo sapiens

<400> 209

```
Leu Arg Leu Val Asp Asp Phe Leu Leu
1               5
```

<210> 210
<211> 9
<212> PRT
<213> Homo sapiens

<400> 210

```
Leu Arg Arg Val Gly Asp Asp Val Leu
1               5
```

<210> 211
<211> 9
<212> PRT
<213> Homo sapiens

<400> 211

```
Tyr Arg Lys Ser Val Trp Ser Lys Leu
1               5
```

<210> 212
<211> 9
<212> PRT
<213> Homo sapiens

<400> 212

```
Gln Arg Leu Cys Glu Arg Gly Ala Lys
1               5
```

<210> 213
<211> 9
<212> PRT
<213> Homo sapiens

<400> 213

```
Phe Arg Ala Leu Val Ala Gln Cys Leu
1               5
```

<210> 214
<211> 9
<212> PRT
<213> Homo sapiens

<400> 214

```
Ser Arg Lys Leu Pro Gly Thr Thr Leu
1               5
```

<210> 215
<211> 9
<212> PRT
<213> Homo sapiens

<400> 215

```
Leu Arg Arg Leu Val Pro Pro Gly Leu
1               5
```

<210> 216
<211> 9
<212> PRT
<213> Homo sapiens

<400> 216

```
Arg Arg Ser Pro Gly Val Gly Cys Val
1               5
```

<210> 217
<211> 9
<212> PRT
<213> Homo sapiens

<400> 217

```
Arg Arg Val Gly Asp Asp Val Leu Val
1               5
```

<210> 218
<211> 9
<212> PRT
<213> Homo sapiens

<400> 218

```
Val Arg Gly Cys Ala Trp Leu Arg Arg
1               5
```

<210> 219
<211> 9
<212> PRT
<213> Homo sapiens

<400> 219

```
Val Arg Ser Leu Leu Arg Ser His Tyr
1               5
```

<210> 220

<211> 9
<212> PRT
<213> Homo sapiens

<400> 220

```
Gly Asp Met Glu Asn Lys Leu Phe Ala
1               5
```

<210> 221
<211> 9
<212> PRT
<213> Homo sapiens

<400> 221

```
Ser Arg Ser Leu Pro Leu Pro Lys Arg
1               5
```

<210> 222
<211> 9
<212> PRT
<213> Homo sapiens

<400> 222

```
Ile Arg Ala Ser Leu Thr Phe Asn Arg
1               5
```

<210> 223
<211> 9
<212> PRT
<213> Homo sapiens

<400> 223

```
Leu Arg Glu Glu Ile Leu Ala Lys Phe
1               5
```

<210> 224
<211> 9
<212> PRT
<213> Homo sapiens

<400> 224

```
Ile Arg Arg Asp Gly Leu Leu Leu Arg
1               5
```

<210> 225
<211> 9
<212> PRT
<213> Homo sapiens

<400> 225

```
                        Gln Arg Gly Asp Pro Ala Ala Phe Arg
                        1               5
```

<210> 226
<211> 9
<212> PRT
<213> Homo sapiens


<400> 226

```
                        Cys Glu Arg Gly Ala Lys Asn Val Leu
                        1               5
```

<210> 227
<211> 9
<212> PRT
<213> Homo sapiens


<400> 227

```
                        Ala Arg Arg Leu Val Glu Thr Ile Phe
                        1               5
```

<210> 228
<211> 9
<212> PRT
<213> Homo sapiens


<400> 228

```
                        Asp Asp Val Leu Val His Leu Leu Ala
                        1               5
```

<210> 229
<211> 9
<212> PRT
<213> Homo sapiens


<400> 229

```
                        Leu Arg Pro Ser Leu Thr Gly Ala Arg
                        1               5
```

<210> 230
<211> 9
<212> PRT
<213> Homo sapiens


<400> 230

```
                        Leu Arg Leu Lys Cys His Ser Leu Phe
                        1               5
```

<210> 231
<211> 9
<212> PRT
<213> Homo sapiens


```
                        Gln Arg Gly Asp Pro Ala Ala Phe Arg
                        1               5
```

<400> 231

```
            Phe Arg Arg Glu Lys Arg Ala Glu Arg
            1               5
```

<210> 232
<211> 9
<212> PRT
<213> Homo sapiens

<400> 232

```
            Ala Arg Gly Gly Pro Pro Glu Ala Phe
            1               5
```

<210> 233
<211> 9
<212> PRT
<213> Homo sapiens

<400> 233

```
            Cys Arg Ala Val Arg Ser Leu Leu Arg
            1               5
```

<210> 234
<211> 9
<212> PRT
<213> Homo sapiens

<400> 234

```
            Gly Arg Thr Arg Gly Pro Ser Asp Arg
            1               5
```

<210> 235
<211> 9
<212> PRT
<213> Homo sapiens

<400> 235

```
            Arg Arg Arg Leu Gly Cys Glu Arg Ala
            1               5
```

<210> 236
<211> 9
<212> PRT
<213> Homo sapiens

<400> 236

```
            Leu Arg Glu Leu Ser Glu Ala Glu Val
            1               5
```

<210> 237
<211> 9
<212> PRT

<213> Homo sapiens

<400> 237

```
Ala Arg Cys Ala Leu Phe Val Leu Val
1               5
```

<210> 238
<211> 9
<212> PRT
<213> Homo sapiens

<400> 238

```
Arg Pro Ala Glu Glu Ala Thr Ser Leu
1               5
```

<210> 239
<211> 9
<212> PRT
<213> Homo sapiens

<400> 239

```
Asp Pro Arg Arg Leu Val Gln Leu Leu
1               5
```

<210> 240
<211> 9
<212> PRT
<213> Homo sapiens

<400> 240

```
Arg Pro Ser Phe Leu Leu Ser Ser Leu
1               5
```

<210> 241
<211> 9
<212> PRT
<213> Homo sapiens

<400> 241

```
Leu Pro Ser Glu Ala Val Gln Trp Leu
1               5
```

<210> 242
<211> 9
<212> PRT
<213> Homo sapiens

<400> 242

```
Tyr Glu Arg Ala Arg Arg Pro Gly Leu
1               5
```

<210> 243

<211> 9
<212> PRT
<213> Homo sapiens

<400> 243

```
Leu Pro Ser Asp Phe Lys Thr Ile Leu
1                   5
```

<210> 244
<211> 9
<212> PRT
<213> Homo sapiens

<400> 244

```
Arg Pro Pro Pro Ala Ala Pro Ser Phe
1                   5
```

<210> 245
<211> 9
<212> PRT
<213> Homo sapiens

<400> 245

```
Leu Pro Arg Leu Pro Gln Arg Tyr Trp
1                   5
```

<210> 246
<211> 9
<212> PRT
<213> Homo sapiens

<400> 246

```
Leu Pro Asn Thr Val Thr Asp Ala Leu
1                   5
```

<210> 247
<211> 9
<212> PRT
<213> Homo sapiens

<400> 247

```
Leu Pro Gly Thr Thr Leu Thr Ala Leu
1                   5
```

<210> 248
<211> 9
<212> PRT
<213> Homo sapiens

<400> 248

```
Leu Ala Lys Phe Leu His Trp Leu Met
1               5
```

<210> 249
<211> 9
<212> PRT
<213> Homo sapiens

<400> 249

```
Lys Ala Lys Asn Ala Gly Met Ser Leu
1               5
```

<210> 250
<211> 9
<212> PRT
<213> Homo sapiens

<400> 250

```
Gly Ser Arg His Asn Glu Arg Arg Phe
1               5
```

<210> 251
<211> 9
<212> PRT
<213> Homo sapiens

<400> 251

```
Lys Ala Leu Phe Ser Val Leu Asn Tyr
1               5
```

<210> 252
<211> 9
<212> PRT
<213> Homo sapiens

<400> 252

```
Ser Pro Leu Arg Asp Ala Val Val Ile
1               5
```

<210> 253
<211> 9
<212> PRT
<213> Homo sapiens

<400> 253

```
Arg Ala Gln Asp Pro Pro Pro Glu Leu
1               5
```

<210> 254
<211> 9
<212> PRT
<213> Homo sapiens

<400> 254

```
                Met Pro Ala His Gly Leu Phe Pro Trp
                1                   5
```

**Description**

This invention relates to peptides which elicit T cell mediated immunity for anti-cancer treatment

Cancer develops through a multistep process involving several mutational events. These mutations result in altered expression/function of genes belonging to two categories: oncogenes and tumour suppressor genes. Oncogenes arise in nature from proto-oncogenes through point mutations or translocations, thereby resulting in a transformed state of the cell harbouring the mutation. All oncogenes code for and function through a protein. Proto-oncogenes are normal genes of the cell which have the potential of becoming oncogenes. In the majority of cases, proto-oncogenes have been shown to be components of signal transduction pathways. Oncogenes act in a dominant fashion. Tumour-suppressor genes on the other hand, act in a recessive fashion, i.e. through loss of function, and contribute to oncogenesis when both alleles encoding the functional protein have been altered to produce non-functional gene products.

The concerted action of a combination of altered oncogenes and tumour-suppressor genes results in cellular transformation and development of a malignant phenotype.

Such cells are however prone to senescence and have a limited life-span. In the majority of cancers, immortalisation of the tumour cells requires the turning on of an enzyme complex called telomerase. In somatic cells the catalytic subunit of this enzyme is normally not expressed. Additional events, such as the action of proteins encoded by a tumour virus or demethylation of silenced promoter sites can result in expression of a functional telomerase complex in tumour cells.

In the field of human cancer immunology, the last two decades have seen intensive efforts to characterise genuine cancer specific antigens. In particular, effort has been devoted to the analysis of antibodies to human tumour antigens. The prior art suggests that such antibodies can be used for diagnostic and therapeutic purposes, for instance in connection with an anti-cancer agent. However, antibodies can only bind to tumour antigens that are exposed on the surface of tumour cells. For this reason, the efforts to produce a cancer treatment based on the immune system of the body has been less successful than expected.

A fundamental feature of the immune system is that it can distinguish self from nonself and does not normally react against self molecules. It has been shown that rejection of tissues or organs grafted from other individuals is an immune response to the foreign antigens on the surface of the grafted cells. The immune response in general consists of a humeral response, mediated by antibodies, and a cellular response. Antibodies are produced and secreted by B lymphocytes, and typically recognise free antigen in native conformation. They can therefore potentially recognise almost any site exposed on the antigen surface. In contrast to antibodies, T cells, which mediate the cellular arm of the immune response, recognise antigens only in the context of MHC molecules, and only after appropriate antigen processing. This antigen processing usually consists of proteolytic fragmentation of the protein, resulting in peptides that fit into the groove of the MHC molecules. This enables T cells to also recognise peptides derived from intracellular antigens.

T cells can recognise aberrant peptides derived from anywhere in the tumour cell, in the context of MHC molecules on the surface of the tumour cell. The T cells can subsequently be activated to eliminate the tumour cell harbouring the aberrant peptide. In experimental models involving murine tumours it has been shown that point mutations in intracellular "self" proteins may give rise to tumour rejection antigens, consisting of peptides differing in a single amino acid from the normal peptide. The T cells recognising these peptides in the context of the major histocompatibility (MHC) molecules on the surface of the tumour cells are capable of killing the tumour cells and thus rejecting the tumour from the host (Boon et al.,1989, Cell 58, 293-303).

MHC molecules in humans are normally referred to as HLA (human leucocyte associated antigen) molecules. There are two principal classes of HLA molecules, class I and class II. HLA class I molecules are encoded by HLA A, B and C subloci and primarily activate CD8+ cytotoxic T cells. HLA class II molecules, on the other hand, primarily activate CD4+ T cells, and are encoded by the DR, DP and DQ subloci. Every individual normally has six different HLA class I molecules, usually two alleles from each of the three subgroups A, B and C, although in some cases the number of different HLA class I molecules is reduced due to the occurrence of the same HLA allele twice.

The HLA gene products are highly polymorphic. Different individuals express distinct HLA molecules that differ from those found in other individuals. This explains the difficulty of finding HLA matched organ donors in transplantations. The significance of the genetic variation of the HLA molecules in immunobiology is reflected by their role as immune-response genes. Through their peptide binding capacity, the presence or absence of certain HLA molecules governs the capacity of an individual to respond to specific peptide epitopes. As a consequence, HLA molecules determine resistance or susceptibility to disease.

T cells may inhibit the development and growth of cancer by a variety of mechanisms. Cytotoxic T cells, both HLA class I restricted CD8+ and HLA class II restricted CD4+ may directly kill tumour cells presenting the appropriate tumour antigens. Normally, CD4+ helper T cells are needed for cytotoxic CD8+ T cell responses, but if the peptide antigen is presented by an appropriate APC, cytotoxic CD8+ T cells can be activated directly, which results in a quicker, stronger and more efficient response.

While the peptides that are presented by HLA class II molecules are of varying length (12-25 amino acids), the peptides presented by HLA class I molecules must normally be exactly nine amino acid residues long in order to fit into the class I HLA binding groove. A longer peptide will result in non-binding if it cannot be processed internally by an APC or target cell, such as a cancer cell, before presenting in the class I HLA groove. Only a limited number of deviations from this requirement of nine amino acids have been reported, and in those cases the length of the presented peptide has been either eight or ten amino acid residues long.

Reviews of how MHC binds peptides can be found in Hans-Georg Rammensee, Thomas Friede and Stefan Stevanovic, (1995, Immunogenetics, 41, 178-228) and in Barinaga (1992, Science 257, 880-881). Male et al (1987, Advanced Immunology, J.B. Lippincott Company, Philadelphia) offers a more comprehensive explanation of the technical background to this invention.

In our International Application PCT/N092/00032 (published as WO92/14756), we described synthetic peptides and fragments of oncogene protein products which have a point of mutation or translocations as compared to their proto-oncogene or tumour suppressor gene protein. These peptides correspond to, completely cover or are fragments of the processed oncogene protein fragment or tumour suppressor gene fragment as presented by cancer cells or other antigen presenting cells, and are presented as a HLA-peptide complex by at least one allele in every individual. These peptides were also shown to induce specific T cell responses to the actual oncogene protein fragment produced by the cell by processing and presented in the HLA molecule. In particular, we described peptides derived from the p21-*ras* protein which had point mutations at particular amino acid positions, namely positions 12, 13 and 61. These peptides have been shown to be effective in regulating the growth of cancer cells *in vitro.* Furthermore, the peptides were shown to elicit CD4+ T cell immunity against cancer cells harbouring the mutated p21-ras oncogene protein through the administration of such peptides in vaccination or cancer therapy schemes. Later we have shown that these peptides also elicit CD8+ T cell immunity against cancer cells harbouring the mutated p21 *ras* oncogene protein through the administration mentioned above (see M.K. Gjertsen et al., Int. J cancer, 1997, vol. 72 p. 784).

However, the peptides described above will be useful only in certain numbers of cancers, namely those which involve oncogenes with point mutations or translocation in a proto-oncogene or tumour suppressor gene. There is therefore a strong need for an anticancer treatment or vaccine which will be effective against a more general range of cancers.

In general, tumours are very heterogeneous with respect to genetic alterations found in the tumour cells. This implies that both the potential therapeutic effect and prophylactic strength of a cancer vaccine will increase with the number of targets that the vaccine is able to elicit T cell immunity against. A multiple target vaccine will also reduce the risk of new tumour formation by treatment escape variants from the primary tumour.

The enzyme telomerase has recently been the focus of attention for its supposed role in prevention of cellular ageing. Telomerase is a RNA-dependent DNA polymerase, which synthesises telomeric DNA repeats using an RNA template that exists as a subunit of the telomerase holoenzyme. The DNA repeats synthesised by the enzyme are incorporated into telomeres, which are specialised DNA-protein structures found at the ends of the linear DNA molecules which make up every chromosome. Telomerase was first identified in the ciliate *Tetrahymena* (Greider and Blackburn, 1985, Cell 43, 405-413). A human telomerase catalytic subunit sequence was recently identified by Meyerson et al (1990, Cell 1197 , 785-795), and Nakamura et al (1997, Science 277, 955-959), who respectively named the gene hEST2 and hTRT. In addition, three other proteins which are associated with telomerase activity have also been identified: p80 and p95 of *Tetrahymena* (Collins et al, 1995, Cell 81, 677-686) and TP1/TLP1, which is the mammalian homologue of *Tetrahymena* p80 (Harrington et al, 1997, Science, 275, 973-977; Nakayama et al., 1997, Cell 88, 875-884).

Telomerase is not expressed in most normal cells in the body. Most somatic lineages in humans show no detectable telomerase activity, but telomerase activity is detected the germline and in some stem cell compartments, which are sites of active cell division (Harley et al., 1994, Cold Spring Harbor Symp. Quant. Biol. 59, 307-315; Kim et al., 1994, Science 266, 2011-2015; Broccoli et al, 1995, PNAS USA 92, 9082-9086; Counter et al., 1995, Blood 85, 2315-2320; Hiyama et al., 1995, J. Immunol. 155, 3711-3715). Telomeres of most types of human somatic cells shorten with increasing age of the organism, consistent with lack of telomerase activity in these cells. Cultured human cells also show telomere shortening. Telomere shortening continues in cultured human cells which have been transformed, until the telomeres have become critically short. At this point, termed the crisis point, significant levels of cell death and karyotypic instability are observed.

Immortal cells, which have acquired the ability to grow indefinitely in culture, emerge at rare frequency from crisis populations. These immortal cells have high levels of telomerase activity and stable telomeres. Telomerase activity is also readily detected in the great majority of human tumour samples analysed to date (Kim et al, 1994, Science 266, 2011-2015), including ovarian carcinoma (Counter et al., 1994, PNAS USA 91, 2900-2904). A comprehensive review is provided by Shay and Bachetti (1997, Eur. J. Cancer 33, 787-791). Thus, activation of telomerase may overcome the barriers to continuous cell division imposed by telomere length. Cells that overcome the normal senescence mechanisms may do so by stabilising telomere length, probably due to the activity of telomerase.

Viruses implicated in human cancer development such as Epstein Barr virus (EBV, related to B cell malignancies and nasopharyngeal carcinomas) and Human Papilloma virus (HPV 16 and 18, related to cervical carcinomas) have

EP 2 316 476 B1

long been known to have the capacity to immortalize human cells. It has now been demonstrated that induction of telomerase activity is the key element in this process (Klingelhutz et al, 1996, Nature, 380, 79-82).

Telomerase is therefore a potential target for cancer therapy. Thus, telomerase inhibitors have been proposed as a new class of anti-cancer drugs (reviewed in Sharma et al, 1997, Ann Oncol 8(11), 1063-1074; Axelrod, 1996, Nature Med 2(2), 158-159; Huminiecki, 1996, Acta Biochim Pol, 43(3), 531-538). It has been suggested that the identification of a human telomerase catalytic subunit may provide a biochemical reagent for identifying such drugs (Meyerson et al, 1990, Cell 1197, 785-795). Telomerase has also been suggested to be a marker for diagnosis or prognosis of cancer (Soria and Rixe, 1997, Bull Cancer 84(10), 963-970; Dahse et al, 1997, Clin Chem 43(5), 708-714).

As far as we are aware, however, no one has previously suggested that telomerase may function as a useful target for T cell mediated therapy, or that telomerase peptides or proteins may be used for the treatment or prophylaxis of cancer.

In accordance with one aspect of the invention, we provide a telomerase peptide for use together with a cytokine and/or growth factor in a method of treatment or prophylaxis of cancer in a human individual, wherein the telomerase peptide consists of between 9 and 25 amino acids and comprises the sequence of SEQ ID NO:2, 3, 4, 9, 10 or 12 to 19, or a fragment of SEQ ID NO:2, 3, 4, 9, 10 or 12 to 19, at least 8 amino acids long, the fragment being capable of generating a T cell response, and wherein the telomerase peptide is administered together, either simultaneously or separately, with the cytokine and/or growth factor in order to strengthen the immune response.

The invention is more particularly described, by way of example only, with reference to the accompanying drawing, in which:

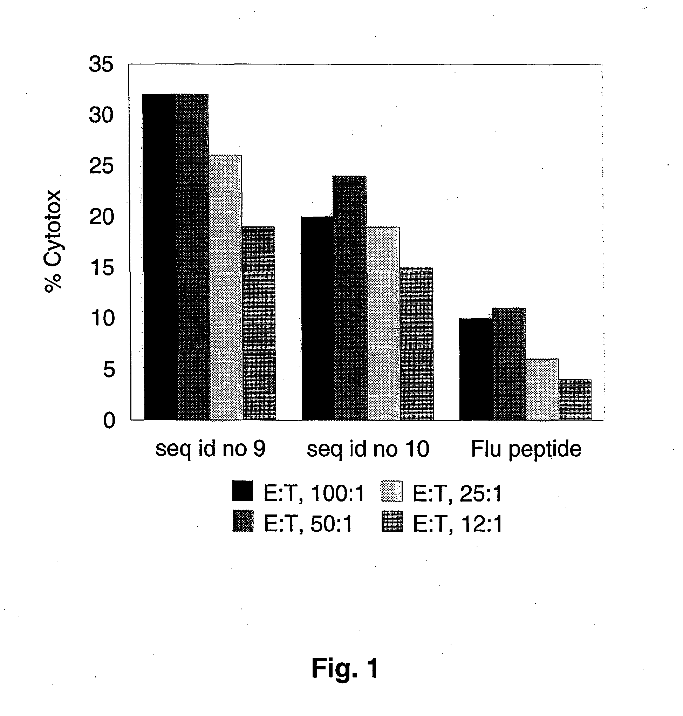

FIGURE 1 shows the sequences of the conserved amino acid motifs in the human telomerase catalytic subunit, as identified by Meyerson et al (1997, Cell 90, 785-795) and Nakamura et al (1997 Science 277, 955-959). Motifs T, 1, 2, 3 (A of Nakamura), 4 (B'of Nakamura) 5 (C of Nakamura), 6 (D of Nakamura) and E are shown. Peptides may be synthesised with sequences corresponding to or encompassing any of the bracketed regions. The designations A2, A1, A3 and B7 indicate peptides which are likely to be presented by HLA-A2, HLA-A1, HLA-A3 and HLA-B7 respectively.

We provide a telomerase peptide for use in a method of treatment or prophylaxis of cancer. In a preferred embodiment, the method comprises generating a T cell response against telomerase. The method may comprise administering to a mammal, preferably a human, suffering or likely to suffer from cancer a therapeutically effective amount of the telomerase peptide so that a T cell response against the telomerase is induced in the mammal.

Telomerase specific T cells may be used to target cells which express telomerase. Thus, since most cells in the body of an organism do not express telomerase, they will be unaffected. However, tumour cells that express telomerase will be targeted and destroyed. As telomerase activity has been detected in the majority of cancers identified so far, we expect our materials and methods to have widespread utility. Cancers which are suitable for treatment include, but are not limited to, breast cancer, prostate cancer, pancreatic cancer, colo-rectal cancer, lung cancer, malignant melanoma, leukaemias, lymphomas, ovarian cancer, cervical cancer and biliary tract carcinomas.

As used here, the term telomerase denotes a ribonucleoprotein enzyme which has telomere elongating activity. Telomerase protein as used here denotes any protein component of telomerase, including any subunit having catalytic activity.

Preferably the telomerase protein is a mammalian telomerase protein, and most preferably a human telomerase protein. The human telomerase protein is preferably the telomerase catalytic subunit identified as hTRT by Nakamura et al (1997, Science 277, 955-959) and hEST2 by Meyerson et al (1990, Cell 1197 , 785-795), the cDNA sequences of which are deposited as GenBank accession numbers AF015950 and AFO18167 respectively.

The term telomerase peptide as used here means a peptide which has an amino acid sequence corresponding to a sequence present in the amino acid sequence of a telomerase protein.

The telomerase peptides preferably contain between 8 and 25 amino acids. More preferably, the telomerase peptides contain between 9 and 25 amino acids. For instance, the telomerase peptides contain 9, 12, 13, 16 or 21 amino acids.

The telomerase peptide is chosen so that it is capable of generating a T cell response directed against the telomerase protein (or against the telomerase protein from which the telomerase peptide is derived). In preferred embodiments, the T cell response induced is a cytotoxic T cell response. The cytotoxic T cell response may be a CD4+ T cell response, or it may be a CD8+ T cell response. In any case, the peptide must be capable of being presented as a complex with a MHC class I or class II protein on the surface of tumour cells or antigen presenting cells, with antigen processing taking place beforehand if necessary.

The telomerase peptide may include one or more amino acid residues from an amino acid motif essential for the biological function of the telomerase protein; in other words, it may overlap at least partially with such an amino acid motif. Examples of such amino acid motifs are motifs 1 to 6 of the human telomerase catalytic subunit sequence hEST2 as identified by Meyerson et al (1990, Cell 1197, 785-795), in other words, from the motifs

LLRSFFYVTE
SRLRFIPK,
LRPIVNMDYVVG,
PELYFVKVDVTGAYDTI,
KSYVQCQGIPQGSILSTLLCSLCY,
LLLRLVDDFLLVT and
GCVVNLRKTVV

or from any of motifs T, 1, 2, A, B', C, D or E as identified by Nakamura et al (1997, Science 277, 955-959) in the hTRT sequence, namely, the motifs

WLMSVYVVELLRSFFYVTETTFQKNRLFFYRKSVWSKLQSIGIRQHLK,
EVRQHREARPALLTSRLRFIPKPDG,
LRPIVNMDYVVGARTFRREKRAERLTSRV,
PPPELYFVKVDVTGAYDTIPQDRLTEVIASIIKP,
KSYVQCQGIPQGSILSTLLCSLCYGDMENKLFAGI,
LLRLVDDFLLVTPHLTH,
AKTFLRTLVRGVPEYGCVVNLRKTVV and HGLFPWCGLLL.

Certain peptides are set out in TABLE 1 as well as in the attached sequence identity list. Another set of peptides derived from elsewhere in the telomerase sequence, are set out in TABLE 2.

Also disclosed are proteins and peptides having amino acid sequences corresponding to an amino acid sequence present in the amino acid sequence of mammalian homologues of the *Tetrahymena* telomerase associated proteins p80 and p95. For example, the p80 homologues TP1 and TLP1 (Harrington et al, 1997, Science, 275, 973-977; Nakayama et al., 1997, Cell 88, 875-884).

Larger peptide fragments carrying a few amino acid substitutions at either the N-terminal end or the C-terminal end are also included, as it has been established that such peptides may give rise to T cell clones having the appropriate specificity.

The peptides described here are particularly suited for use in a vaccine capable of safely eliciting either CD4+ or CD8+ T cell immunity:

(a) the peptides are synthetically produced and therefore do not include transforming cancer genes or other sites or materials which might produce deleterious effects,
(b) the peptides may be used alone to induce cellular immunity,
(c) the peptides may be targeted for a particular type of T cell response without the side effects of other unwanted responses.

The telomerase peptides described here can be administered in an amount in the range of 1 microgram (1μg) to 1 gram (1g) to an average human patient or individual to be vaccinated. It is preferred to use a smaller dose in the range of 1 microgram (1μg) to 1 milligram (1mg) for each administration.

The telomerase peptide can be provided to the patient in the form of a pharmaceutical composition. The telomerase peptide may be administered as a mixture of proteins or a mixture of proteins and peptides or a mixture of peptides. The pharmaceutical composition may in addition include the usual additives, diluents, stabilisers or the like as known in the art.

The pharmaceutical composition may comprise one or more telomerase peptides. The peptide mixture may be any one of the following:

(a) a mixture of peptides having different sequences, for example, corresponding to different portions of a telomerase protein sequence;
(b) a mixture of peptides having overlapping sequences, but suitable to fit different HLA alleles;
(c) a mixture of both mixtures (a) and (b);
(d) a mixture of several mixtures (a);
(e) a mixture of several mixtures (b);
(f) a mixture of several mixtures (a) and several mixtures (b);

In each case, a mixture of proteins or peptides corresponding to different telomerase proteins, for example, a telomerase catalytic subunit and a *Tetrahymena* p80 or p95 homologue, may also be used.

Alternatively, the telomerase peptides in the mixture may be covalently linked with each other to form larger polypeptides or even cyclic polypeptides. The pharmaceutical composition may be made by mixing the telomerase protein(s) or peptide(s) with a pharmaceutically acceptable carrier or diluent.

The pharmaceutical composition may also include at least one peptide capable of inducing a T cell response against an oncogene or mutant tumour suppressor protein or peptide. Alternatively, the telomerase proteins or peptides may be administered either simultaneously or in optional sequence with these peptides. Examples of oncogene proteins are the p21-ras proteins H-ras, K-ras and N-ras, abl, gip, gsp, ret and trk. Preferably, the oncogene protein or peptide is a p21-*ras* protein or peptide, for example, the p21-*ras* peptides described in our International Application PCT/NO92/00032 (publication number WO92/14756). Tumour suppressor proteins include p53 and Rb (retinoblastoma). Such a pharmaceutical composition may be made by mixing the telomerase protein(s) or peptide(s) with the mutant tumour suppressor or oncogene proteins or peptides, together with a pharmaceutically acceptable carrier or diluent.

As used here, the term mutant refers to a wild type sequence which has one or more of the following: point mutation (transition or transversion), deletion, insertion, duplication translocation or inversion. The term pharmaceutical composition not only encompasses a composition usable in treatment of cancer patients, but also includes compositions useful in connection with prophylaxis, i.e., vaccine compositions.

The telomerase peptides are administered to a human individual in need of such treatment or prophylaxis.

The administration may take place one or several times as suitable to establish and/or maintain the wanted T cell immunity. The peptides are administered together, either simultaneously or separately, with cytokines and/or growth factors, i.e., interleukin-2 (IL-2), interleukin-12 (IL-12), granulocyte macrophage colony stimulating factor (GM-CSF) or the like in order to strengthen the immune response as known in the art. The telomerase proteins or peptides can be used in a vaccine or a therapeutical composition either alone or in combination with other materials. For example, the peptide or peptides may be supplied in the form of a lipopeptide conjugate which is known to induce a high-affinity cytotoxic T cell response (Deres, 1989, Nature 342).

We describe a method of treatment of a patient afflicted with cancer, the method comprising eliciting T-cell responses through stimulating *in vivo* or *ex vivo* with a telomerase protein or peptide. The telomerase protein or peptide can also be used in a method of vaccination of a patient in order to obtain resistance against cancer. A suitable method of vaccination comprises eliciting T-cell responses through stimulating *in vivo* or *ex vivo* with a telomerase protein or peptide. We also describe a method of treatment or prophylaxis of cancer, comprising administering to a mammal suffering or likely to suffer from cancer a therapeutically effective amount of a telomerase protein or peptide so that a T cell response against telomerase is induced in the mammal.

The peptides described here may be produced by conventional processes, for example, by the various peptide synthesis methods known in the art. Alternatively, they may be fragments of a telomerase protein produced by cleavage, for example, using cyanogen bromide, and subsequent purification. Enzymatic cleavage may also be used. The telomerase proteins or peptides may also be in the form of recombinant expressed proteins or peptides.

Nucleic acids encoding the telomerase peptide can be made by oligonucleotide synthesis. This may be done by any of the various methods available in the art. A nucleic acid encoding telomerase protein may be cloned from a genomic or cDNA library, using conventional library screening. The probe may correspond to a portion of any sequence of a known telomerase gene. Alternatively, the nucleic acid can be obtained by using the Polymerase Chain Reaction (PCR). The nucleic acid is preferably DNA, and may suitably be cloned into a vector. Subclones may be generated by using suitable restriction enzymes. The cloned or subcloned DNA may be propagated in a suitable host, for example a bacterial host. Alternatively, the host can be a eukaryotic organism, such as yeast or baculovirus. The telomerase protein or peptides may be produced by expression in a suitable host. In this case, the DNA is cloned into an expression vector. A variety of commercial expression kits are available. The methods described in Maniatis et al (1991, Molecular Cloning: A Laboratory Manual, Cold Spring Harbor, New York, Cold Spring Harbor Laboratory Press) and Harlow and Lane (1988, Antibodies: A Laboratory Manual, Cold Spring Harbor, New York, Cold Spring Harbor Laboratory Press) may be used for these purposes.

Experimental Methods

The peptides were synthesised by using continuous flow solid phase peptide synthesis. N-a-Fmoc-amino acids with appropriate side chain protection were used. The Fmoc-amino acids were activated for coupling as pentafluorophenyl esters or by using either TBTU or diisopropyl carbodiimide activation prior to coupling. 20% piperidine in DMF was used for selective removal of Fmoc after each coupling. Cleavage from the resin and final removal of side chain protection was performed by 95% TFA containing appropriate scavengers. The peptides were purified and analysed by reversed phase (C18) HPLC. The identify of the peptides was confirmed by using electro-spray mass spectroscopy (Finnigan mat SSQ720).

In order for a cancer vaccine and methods for specific cancer therapy based on T cell immunity to be effective, three conditions must be met:

> (a) the peptide is at least 8 amino acids long and is a fragment of a telomerase protein and
> (b) the peptide is capable of inducing, either in its full length or after processing by antigen presenting cell, T cell responses.

The following experimental methods may be used to determine if these three conditions are met for a particular peptide. First, it should be determined if the particular peptide gives rise to T cell immune responses *in vitro.* It will also need to be established if the synthetic peptides correspond to, or are capable after processing to yield, peptide fragments corresponding to peptide fragments occurring in cancer cells harbouring telomerase or antigen presenting cells that have processed naturally occurring telomerase. The specificity of T cells induced *in vivo* by telomerase peptide vaccination may also be determined.

It is necessary to determine if telomerase expressing tumour cell lines can be killed by T cell clones obtained from peripheral blood from carcinoma patients after telomerase peptide vaccination. T cell clones are obtained after cloning of T-cell blasts present in peripheral blood mononuclear cells (PBMC) from a carcinoma patient after telomerase peptide vaccination. The peptide vaccination protocol includes several *in vivo* injections of peptides intracutaneously with GM-CSF or another commonly used adjuvant. Cloning of T cells is performed by plating responding T cell blasts at 5 blasts per well onto Terasaki plates. Each well contains $2 \times 10^4$ autologous, irradiated (30 Gy) PBMC as feeder cells. The cells are propagated with the candidate telomerase peptide at 25 mM and 5 U/ml recombinant interleukin-2 (rIL-2) (Amersham, Aylesbury, UK) in a total volume of 20 mL. After 9 days T cell clones are transferred onto flat-bottomed 96-well plates (Costar, Cambridge, MA) with 1 mg/ml phytohemagglutinin (PHA, Wellcome, Dartford, UK), 5 U/ml rIL-2 and allogenic irradiated (30 Gy) PBMC ($2 \times 10^5$) per well as feeder cells. Growing clones are further expanded in 24-well plates with PHA / rIL-2 and $1 \times 10^6$ allogenic, irradiated PBMC as feeder cells and screened for peptide specificity after 4 to 7 days.

T cell clones are selected for further characterisation. The cell-surface phenotype of the T cell clone is determined to ascertain if the T cell clone is CD4+ or CD8+. T cell clone is incubated with autologous tumour cell targets at different effector to target ratios to determine if lysis of tumour cells occurs. Lysis indicates that the T cell has reactivity directed against a tumour derived antigen, for example, telomerase protein.

In order to verify that the antigen recognised is associated with telomerase protein, and to identify the HLA class I or class II molecule presenting the putative telomerase peptide to the T cell clone, different telomerase expressing tumour cell lines carrying one or more HLA class I or II molecules in common with those of the patient are used as target cells in cytotoxicity assays. Target cells are labelled with $^{51}$Cr or $^{3}$H-thymidine ($9.25 \times 10^4$ Bq/mL) overnight, washed once and plated at 5000 cells per well in 96 well plates. T cells are added at different effector to target ratios and the plates are incubated for 4 hours at 37°C and then harvested before counting in a liquid scintillation counter (Packard Topcount). For example, the bladder carcinoma cell line T24 (12Val$^+$, HLA-A1$^+$, B35$^+$), the melanoma cell line FMEX (12Val$^+$, HLA-A2$^+$, B35$^+$) and the colon carcinoma cell line SW 480 (12Val$^+$, HLA-A2$^+$, B8$^+$) or any other telomerase positive tumour cell line may be used as target cells. A suitable cell line which does not express telomerase protein may be used as a control, and should not be lysed. Lysis of a particular cell line indicates that the T cell clone being tested recognises an endogenously-processed telomerase epitope in the context of the HLA class I or class II subtype expressed by that cell line.

The HLA class I or class II restriction of a T cell clone may be determined by blocking experiments. Monoclonal antibodies against HLA class I antigens, for example the panreactive HLA class I monoclonal antibody W6/32, or against class II antigens, for example, monoclonals directed against HLA class II DR, DQ and DP antigens (B8/11, SPV-L3 and B7/21), may be used. The T cell clone activity against the autologous tumour cell line is evaluated using monoclonal antibodies directed against HLA class I and class II molecules at a final concentration of 10 mg/ml. Assays are set up as described above in triplicate in 96 well plates and the target cells are preincubated for 30 minutes at 37°C before addition of T cells.

The fine specificity of a T cell clone may be determined using peptide pulsing experiments. To identify the telomerase peptide actually being recognised by a T cell clone, a panel of nonamer peptides is tested. $^{51}$Cr or $^{3}$H-thymidine labelled, mild acid eluted autologous fibroblasts are plated at 2500 cells per well in 96 well plates and pulsed with the peptides at a concentration of 1 mM together with b2-microglobulin (2.5 mg/mL) in a 5% $CO_2$ incubator at 37°C before addition of the T cells. Assays are set up in triplicate in 96 well plates and incubated for 4 hours with an effector to target ratio of 5 to 1. Controls can include T cell clone cultured alone, with APC in the absence of peptides or with an irrelevant melanoma associated peptide MART-1/Melan-A peptide.

An alternative protocol to determine the fine specificity of a T cell clone may also be used. In this alternative protocol, the TAP deficient T2 cell line is used as antigen presenting cells. This cell line expresses only small amounts of HLA-A2 antigen, but increased levels of HLA class I antigens at the cell surface can be induced by addition of b2-microglobulin. $^{3}$H-labelled target cells are incubated with the different test peptides and control peptides at a concentration of 1 mM together with b2-microglobulin (2.5 mg/mL) for one hour at 37°C. After peptide pulsing, the target cells are washed extensively, counted and plated at 2500 cells per well in 96 well plates before addition of the T cells. The plates are incubated for 4 hours at 37°C in 5% $CO_2$ before harvesting. Controls include T cell clone cultured alone or with target cells in the absence of peptides. Assays were set up in triplicate in 96 well plates with an effector to target ratio of 20 to 1.

The sensitivity of a T cell clone to a particular peptide identified above may also be determined using a dose-response experiment. Peptide sensitised fibroblasts can be used as target cells. The target cells are pulsed with the particular peptide as described above for fine specificity determination, with the exception that the peptides are added at different concentrations before the addition of T cells. Controls include target cells alone and target cells pulsed with the irrelevant melanoma associated peptide Melan-A/Mart-1.

Biological experiments/ Description of the figures:

Figure 1

Figure 1 (Fig. 1) describes the induction of telomerase (hTERT) reactive cytotoxic T lymphocytes (CTL's) in HLA-A2(A2/K$^b$) transgenic mice immunized with telomerase peptides with sequence identity 9 and 10. A standard HLA-A2 restricted influenza (58-66) peptide was used as control. Three groups of five mice each were given two weekly subcutaneous injections of $10^7$ irradiated, peptide pulsed (100 $\mu$g/ml) syngeneic spleen cells. One week after the second injection, the mice were sacrificed and their spleens harvested. Spleen cells were prepared by standard techniques, and cells from primed animals were restimulated in vitro for 5 days by coculture with peptide pulsed (10 $\mu$g/ml) irradiated auatologous spleen cells as antigen presenting cells before testing of cytotoxicity against hTERT expressing target cells (Jurkat) transfected with HLA-A2 (A2/K$^b$) in a $^{51}$Cr release assay.

Columns to the left of Fig. 1 show killing of HLA-A2 transfected Jurkat cells pulsed with the control peptide (influenza 58-66) by T cells obtained after priming of mice with the peptide with sequence identity 9, at different effector to target ratios. Specific cytotoxicity above background was observed at all effector to target ratios. Columns in the middle show similar data with T cells obtained from mice primed with the peptide with sequence identity 10. Significant killing of Jurkat cells was only observed when spleen cells from telomerase peptide pulsed mice were used as effector cells, thus when spleen cells from influenza peptide primed mice were used as effectors, only background level of killing of Jurkat cells was seen when the target cells were pulsed with an irrelevant peptide (melanocortin receptor 1 peptide, MC1R244) as evident from columns in the right part of Fig. 1. These results demonstrate that the peptides with sequence identity 9 and 10 are immunogenic in vivo and upon immunization may elicit an immune response in a warm blooded animal carrying the common human MHC molecule HLA-A2. This finding indicates that the peptides with seq. id. no. 9 and 10 may also be used as a cancer vaccine in humans carrying HLA-A2 and other HLA class I molecules capable of binding these peptides. Furthermore, these results demonstrate that hTERT expressed by the T cell leukemia line Jurkat can be processed by the proteolytic machinery of the cell line to yield peptide fragments identical with or similar to the peptides with sequence identity 9 and 10. Together these observations indicate that an immune response obtained after vaccination of cancer patients or patients at risk of developing cancer with these peptides may result in efficient killing of tumor cells expressing the hTERT subunit of telomerase.

Fig. 1 depicts cytotoxicity of HLA-A2 transfected Jurkat cells with effector cells obtained from mice immunized as indicated in the figure. Target cells were labeled with $^{51}$Cr (0,1 $\mu$Ci/100 $\mu$l cell suspension) for 1 hr. at 37 °C, washed twice and pulsed with peptide (1 $\mu$g/ml) for 1 hr at 37 °C before washing. Two thousand labeled, peptide pulsed target cells were seeded per well in a 96 well v-bottom microtitre plate, and effector cells (from $2,5 \times 10^4$ to $2 \times 10^5$) were added to the wells. Cultures were incubated for 4 hrs. at 37 °C and supernatants were harvested and tested in a gamma-counter. The results in Fig. 1 are expressed as specific cytotoxicity calculated by the following formula:

$$\text{(cpm experimental released – cpm spontaneously released)/}$$
$$\text{(cpm total – cpm spontaneously released) x 100}$$

Figure 2

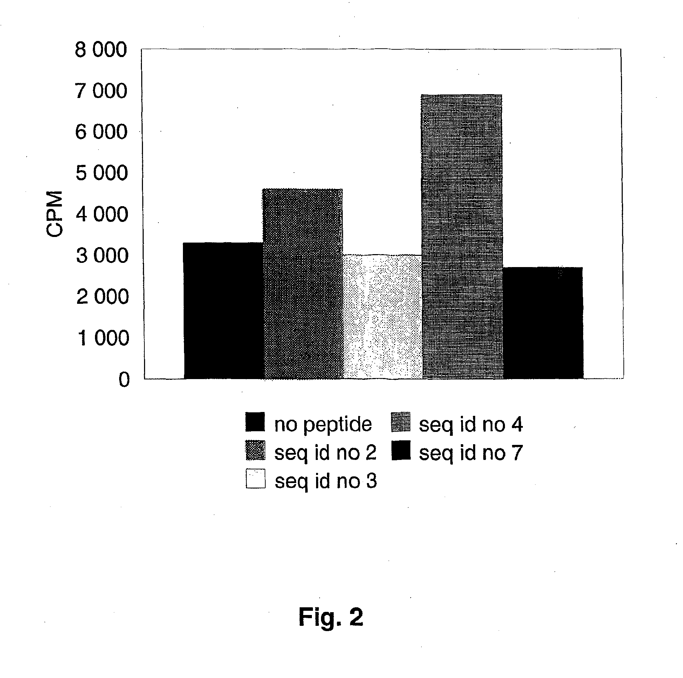

Figure 2 (Fig. 2) shows the results of in vitro stimulation of peripheral blood T cells from a patient (TT) with colon cancer with telomerase (hTERT) derived peptides with sequence identity number 2, 3, 4 and 7. In vitro culture was performed as follows: Triplicates of $10^5$ mononuclear cells were incubated for 6 days in X-VIVO 10 medium supplemented with 15% pooled heat inactivated human serum in a humidified incubator in 5% $CO_2$. Peptides were present throughout culture at a final concentration of 30 $\mu$g/ml in the medium. Cultures without peptide served as control. A proliferative response above background values was seen when the T cells were stimulated with the peptide with sequence identity 4. These results demonstrate that blood from a cancer patient contains circulating T cells specific for a peptide derived from telomerase (hTERT). These results demonstrate that the enzymatic subunit of telomerase (hTERT) is immunogenic in man, and may spontaneously give rise to telomerase specific T cell responses when overexpressed by a tumor growing in the patient. Furthermore, one component of the telomerase specific response in this patient is directed against the peptide with seq. id. no. 4 described here. This finding indicates that the peptide with seq. id. no. 4 may also be used as a cancer vaccine in humans. The figure depicts the results of conventional T cell proliferative assays, where peripheral blood mononuclear cells ($10^5$) were cultured with peptides as indicated for 7 days in triplicates before harvesting. To measure the proliferative capacity of the cultures, $^3$H-thymidine ($3,7\times10^4$ Bq/ well) was added to the culture overnight before harvesting. Values are given as mean counts per minute (cpm) of the triplicates.

Figures 3 and 4

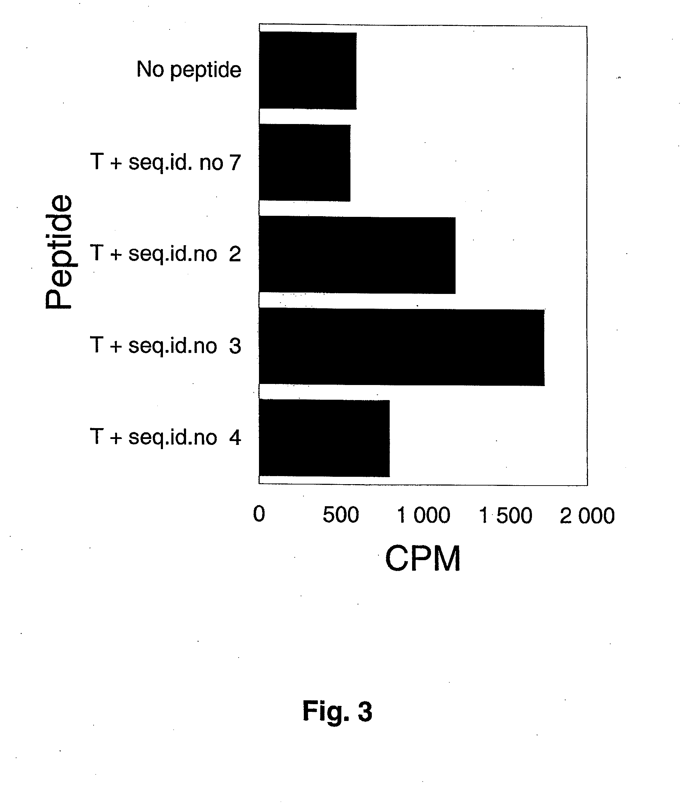

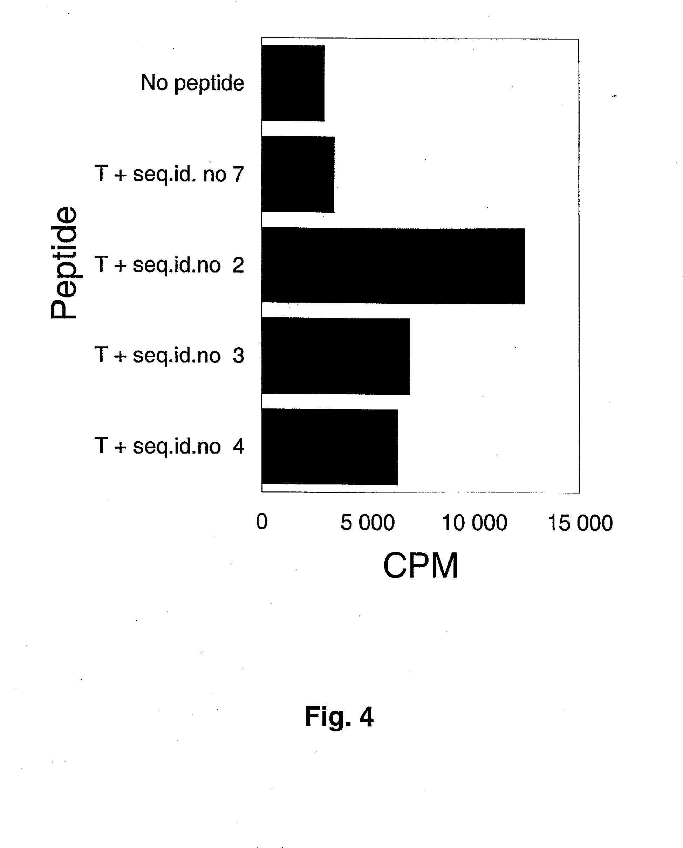

Figures 3 and 4 (Fig. 3 and Fig. 4) show the reactivity of tumor infiltrating lymphocytes (TILs) obtained from a patient with advanced pancreatic cancer. The T cells were obtained from a tumor biopsy and was successfully propagated *in vitro* to establish a T cell line. The T cell line was CD3+, CD4+ and CD8-, and proliferated specificially in response to the telomerase peptides. The results in Fig. 3 show T cells that recognise the peptides with seq. id. no. 2 and 3 when compared to controls with medium alone. The results in Fig. 4 show T cells that recognise the peptide with seq. id. no. 2. The TILs were expanded by co-cultureing with recombinant human interleukin 2 (rIL-2) and tested after 14 days in standard proliferation assay using peptides with sequence id. nos. 2, 3, 4 and 7.

Table 1

| | |
|---|---|
| LMSVYVVEL | FLHWLMSVYVVELLRSFFYVTE |
| ELLRSFFYV | EARPALLTSRLRFIPK |
| YVVELLRSF | DGLRPIVNMDYVVGAR |
| WELLRSFF | GVPEYGCVVNLRKVVNF |
| SVYVVELLR | |
| VELLRSFFY | |
| YVTETTFQK | |
| RLFFYRKSV | |
| SIGIRQHLK | |
| RPALLTSRL | |
| ALLTSRLRF | |
| LLTSRLRFI | |
| RPIVNMDYV | |
| LRPIVNMDY | |
| YVVGARTFR | |
| VVGARTFRR | |
| GARTFRREK | |
| ARTFRREKP | |
| PPELYFVKV | |
| ELYFVKVDV | |
| FVKVDVTGA | |
| IPQDRLTEV | |
| DRLTEVIAS | |
| RLTEVIASI | |
| IPQGSILSTL | |
| ILSTLLCSL | |
| LLRLVDDFL | |
| RLVDDFLLV | |
| VPEYGCVVN | |
| VPEYGCVVNL | |
| TLVRGVPEY | |
| FLRTLVRGV | |
| GVPEYGCVV | |

(continued)

VVNLRKTVV
GLFPWCGLL

Table 2

YAETKHFLY
ISDTASLCY
DTDPRRLVQ
AQDPPPELY
LTDLQPYMR
QSDYSSYAR

ILAKFLHWL
ELLRSFFYV
LLARCALFV
WLCHQAFLL
RLVDDFLLV
RLFFYRKSV
LQLPFHQQV
RLGPQGWRL
SLQELTWKM
NVLAFGFAL
VLLKTHCPL
FLLVTPHLT
TLTDLQPYM
RLTEVIASI
FLDLQVNSL
SLNEASSGL
ILSTLLCSL
LLGASVLGL
VLAFGFALL
LQPYMRQFV
LMSVYVVEL
RLPQRYWQM
RQHSSPWQV
YLPNTVTDA
NMRRKLFGV
RLTSRVKAL
LLQAYRFHA
LLDTRTLEV
YMRQFVAHL
LLTSRLRFI
CLVCVPWDA
LLSSLRPSL
FMCHHAVRI
LQVNSLQTV
LVAQCLVCV
CLKELVARV
FLRNTKKFI
ALPSDFKTI
VLVHLLARC

(continued)

VQSDYSSYA
SVWSKLQSI
KLPGTTLTA
QLSRKLPGT
ELYFVKVDV
GLLLDTRTL
WMPGTPRRL
SLTGARRLV
VVIEQSSSL
LPSEAVQWL
QAYRFHACV

GLFDVFLRF
KLFGVLRLK
RLREEILAK
TLVRGVPEY
GLPAPGARR
GLFPWCGLL
KLTRHRVTY
VLPLATFVR
ELVARVLQR

DPRRLVQLL
FVRACLRRL
SVREAGVPL
AGRNMRRKL
LARCALFVL
RPAEEATSL
LPSDFKTIL
LPSEAVQWL
LPGTTLTAL
RPSFLLSSL
LPNTVTDAL
RPALLTSRL
RCRAVRSLL
MPRAPRCRA

GIRRDGLLL
VLRLKCHSL
YMRQFVAHL
SLRTAQTQL
QMRPLFLEL
LLRLVDDFL
FVQMPAHGL
HASGPRRRL
VVIEQSSSL
RVISDTASL
CVPAAEHRL
RVKALFSVL
NVLAFGFAL
LVARVLQRL

(continued)

FAGIRRDGL
HAQCPYGVL
RAQDPPPEL
AYRFHACVL
HAKLSLQEL
GAKGAAGPL
TASLCYSIL
APRCRAVRS
GARRLVETI
AQCPYGVLL
HAKTFLRTL
EATSLEGAL
KAKNAGMSL
AQTQLSRKL
AGIRRDGLL

VLRLKCHSL
ILKAKNAGM
DPRRLVQLL
GAKGAAGPL
FAGIRRDGL
GARRRGGSA
HAKTFLRTL
HAKLSLQEL
LARCALFVL
EHRLREEIL
NMRRKLFGV

CAREKPQGS
LTRHRVTYV

RRFLRNTKK
RRDGLLLRL
RREKRAERL
RRLVETIFL
LRFMCHHAV
RRYAVVQKA
KRAERLTSR
RRKLFGVLR
RRRGGSASR
RRLPRLPQR
RRLGPQGWR
LRGSGAWGL
HREARPALL
VRRYAVVQK
ARTSIRASL
HRVTYVPLL
LRSHYREVL
MRPLFLELL
HRAWRTFVL
MRRKLFGVL

(continued)

LRLVDDFLL
LRRVGDDVL
YRKSVWSKL
QRLCERGAK
FRALVAQCL
SRKLPGTTL
LRRLVPPGL
RRSPGVGCV
RRVGDDVLV
VRGCAWLRR
VRSLLRSHY
ARTFRREKR
SRSLPLPKR
IRASLTFNR
LREEILAKF
IRRDGLLLR
QRGDPAAFR
LRPIVNMDY

ARRLVETIF
ARPALLTSR
LRPSLTGAR
LRLKCHSLF
FRREKRAER
ARGGPPEAF
CRAVRSLLR
GRTRGPSDR
RRRLGCERA
LRELSEAEV
ARCALFVLV

RPAEEATSL
DPRRLVQLL
RPSFLLSSL
LPSEAVQWL
RPALLTSRL
LPSDFKTIL
RPPPAAPSF
LPRLPQRYW
LPNTVTDAL
LPGTTLTAL
LAKFLHWLM
KAKNAGMSL
GSRHNERRF
KALFSVLNY
SPLRDAVVI
RAQDPPPEL
MPAHGLFPW

AEVRQHREA
REAGVPLGL

(continued)

EEATSLEGA
LEAAANPAL
QETSPLRDA
REVLPLATF
KEQLRPSFL
REKPQGSVA
LEVQSDYSS
REARPALLT
EEDTDPRRL
REEILAKFL
CERGAKNVL
DDVLVHLLA
GDMENKLFA
YERARRPGL

**Claims**

1. A telomerase peptide for use together with a cytokine and/or growth factor in a method of treatment or prophylaxis of cancer in a human individual, wherein the telomerase peptide consists of between 9 and 25 amino acids and comprises the sequence of SEQ ID NO:2, 3, 4, 9, 10 or 12 to 19, or a fragment of SEQ ID NO:2, 3, 4, 9, 10 or 12 to 19, at least 8 amino acids long, the fragment being capable of generating a T cell response, and wherein the telomerase peptide is administered together, either simultaneously or separately, with the cytokine and/or growth factor in order to strengthen the immune response.

2. A telomerase peptide for use as claimed in claim 1, the telomerase peptide being capable of generating a T cell response directed against the telomerase protein.

3. A telomerase peptide for use as claimed in claim 1 or 2, in which the method comprises administering to a mammal suffering or likely to suffer from cancer a therapeutically effective amount of the telomerase protein or peptide so that a T cell response against the telomerase is induced in the mammal.

4. A telomerase peptide for use as claimed in any of the preceding claims, in which the T cell response induced is a cytotoxic T cell response.

5. A telomerase peptide for use as claimed in any of the preceding claims, in which the telomerase peptide is a human telomerase peptide.

6. A telomerase peptide for use as claimed in any of the preceding claims, in which the telomerase peptide has a length of 9, 12, 13, 16 or 21 amino acids.

7. A telomerase peptide for use as claimed in any of Claims 1 to 5, in which the peptide has a length of at least 9 amino acids.

8. A telomerase peptide for use as claimed in any of the preceding claims, in which the telomerase peptide consists of the sequence of SEQ ID NO:2, 3, 4, 9, 10 or 12 to 19.

9. A telomerase peptide for use as claimed in any of the preceding claims, in which the cytokine or growth factor is selected from the group consisting of interleukin-2 (IL-2), interleukin-12 (IL-12) and granulocyte macrophage colony stimulating factor (GM-CSF).

10. A telomerase peptide for use as claimed in any of the preceding claims, in which the cytokine or growth factor is GM-CSF.

11. A telomerase peptide for use as claimed in any of the preceding claims, in which the telomerase peptide is administered several times.

12. A telomerase peptide for use as claimed in any of the preceding claims, in which the telomerase peptide is administered intracutaneously with GM-CSF.

13. A telomerase peptide for use as claimed in any of the preceding claims wherein the cancer is selected from the group consisting of breast cancer, prostate cancer, pancreatic cancer, colo-rectal cancer, lung cancer, malignant melanoma, leukaemias, lymphomas, ovarian cancer, cervical cancer and biliary tract carcinomas.

**Patentansprüche**

1. Telomerasepeptid zur gemeinsamen Verwendung mit einem Cytokin und/oder einem Wachstumsfaktor in einem Verfahren zur Behandlung von Krebs oder zur Krebsprophylaxe in einem menschlichen Individuum, wobei das Telomerasepeptid aus 9 bis 25 Aminosäuren besteht und die Sequenz der SEQ ID Nr.: 2, 3, 4, 9, 10 oder 12 bis 19 umfaßt, oder ein Fragment der SEQ ID Nr.: 2, 3, 4, 9, 10 oder 12 bis 19 mit einer Länge von zumindest 8 Aminosäuren, wobei das Fragment dazu fähig ist, eine T-Zellen-Antwort zu induzieren, und wobei das Telomerasepeptid zusammen, entweder gleichzeitig oder separat, mit einem Cytokin und/oder einem Wachstumsfaktor verabreicht wird, um die Immunantwort zu verstärken.

2. Telomerasepeptid zur Verwendung gemäß Anspruch 1, wobei das Telomerasepeptid dazu fähig ist, eine gegen das Telomeraseprotein gerichtete T-Zellen-Antwort hervorzurufen.

3. Telomerasepeptid zur Verwendung gemäß Anspruch 1 oder 2, wobei das Verfahren das Verabreichen einer therapeutisch wirksamen Menge des Telomeraseproteins oder -peptids an ein Säugetier umfaßt, welches an Krebs erkrankt oder wahrscheinlich an Krebs erkrankt ist, so daß in dem Säugetier eine T-Zellen-Antwort induziert wird, die gegen die Telomerase gerichtet ist.

4. Telomerasepeptid zur Verwendung gemäß einem der vorhergehenden Ansprüche, wobei die hervorgerufene T-Zellen-Antwort eine cytotoxische T-Zellen-Antwort ist.

5. Telomerasepeptid zur Verwendung gemäß einem der vorhergehenden Ansprüche, wobei das Telomerasepeptid ein menschliches Telomerasepeptid ist.

6. Telomerasepeptid zur Verwendung gemäß einem der vorhergehenden Ansprüche, wobei das Telomerasepeptid eine Länge von 9, 12, 13, 16 oder 21 Aminsäuren aufweist.

7. Telomerasepeptid zur Verwendung gemäß einem der Ansprüche 1 bis 5, wobei das Telomerasepeptid eine Länge von zumindest 9 Aminsäuren aufweist.

8. Telomerasepeptid zur Verwendung gemäß einem der vorhergehenden Ansprüche, wobei das Telomerasepeptid aus der Sequenz der SEQ ID Nr.: 2, 3, 4, 9, 10 oder 12 bis 19 besteht.

9. Telomerasepeptid zur Verwendung gemäß einem der vorhergehenden Ansprüche, wobei das Cytokin oder der Wachstumsfaktor ausgewählt sind aus der Gruppe bestehend aus Interleukin-2 (IL-2), Interleukin-12 (IL-12) und dem Granulozyten-Makrophagen-Kolonie-stimulierenden Faktor (GM-CSF).

10. Telomerasepeptid zur Verwendung gemäß einem der vorhergehenden Ansprüche, wobei das Cytokin oder der Wachstumsfaktor GM-CSF ist.

11. Telomerasepeptid zur Verwendung gemäß einem der vorhergehenden Ansprüche, wobei das Telomerasepeptid mehrmals verabreicht wird.

12. Telomerasepeptid zur Verwendung gemäß einem der vorhergehenden Ansprüche, wobei das Telomerasepeptid intrakutan mit GM-CSF verabreicht wird.

13. Telomerasepeptid zur Verwendung gemäß einem der vorhergehenden Ansprüche, wobei der Krebs ausgewählt ist aus der Gruppe bestehend aus Brustkrebs, Prostatakrebs, Pankreaskrebs, kolorektalem Krebs, Lungenkrebs, malignem Melanom, Leukämien, Lymphomen, Eierstockkrebs, Gebärmutterhalskrebs und Gallenwegkarzinomen.

**Revendications**

1. Peptide de la télomérase destiné à être utilisé ensemble avec une cytokine et/ou un facteur de croissance dans un procédé de traitement ou de prophylaxie du cancer chez un être humain, dans lequel le peptide de la télomérase est constitué de 9 à 25 acides aminés et comprend la séquence SEQ ID n°: 2, 3, 4, 9, 10 ou 12 à 19, ou un fragment de la SEQ ID n°: 2, 3, 4, 9, 10 ou 12 à 19, d'une longueur d'au moins 8 acides aminés, le fragment étant capable de générer une réponse de la cellule T, et dans lequel le peptide de la télomérase est administré ensemble, soit simultanément, soit séparément, avec la cytokine et/ou le facteur de croissance pour renforcer la réponse immunitaire.

2. Peptide de la télomérase destiné à être utilisé selon la revendication 1, le peptide de la télomérase étant capable de générer une réponse de la cellule T dirigée contre la protéine de la télomérase.

3. Peptide de la télomérase destiné à être utilisé selon la revendication 1 ou 2, dans lequel le procédé comprend le fait d'administrer à un mammifère souffrant ou susceptible de souffrir de cancer une quantité thérapeutiquement efficace de la protéine ou du peptide de télomérase de sorte qu'une réponse de la cellule T contre la télomérase soit induite chez le mammifère.

4. Peptide de la télomérase destiné à être utilisé selon l'une quelconque des revendications précédentes, dans laquelle la réponse de la cellule T induite est une réponse de la cellule T cytotoxique.

5. Peptide de la télomérase destiné à être utilisé selon l'une quelconque des revendications précédentes, dans lequel le peptide de la télomérase est un peptide de la télomérase humaine.

6. Peptide de la télomérase destiné à être utilisé selon l'une quelconque des revendications précédentes, dans lequel le peptide de la télomérase présente une longueur de 9, 12, 13, 16 ou 21 acides aminés.

7. Peptide de la télomérase destiné à être utilisé selon l'une quelconque des revendications 1 à 5, dans lequel le peptide présente une longueur d'au moins 9 acides aminés.

8. Peptide de la télomérase destiné à être utilisé selon l'une quelconque des revendications précédentes, dans lequel le peptide de la télomérase se compose de la séquence SEQ ID n°: 2, 3, 4, 9, 10 ou 12 à 19.

9. Peptide de la télomérase destiné à être utilisé selon l'une quelconque des revendications précédentes, dans lequel la cytokine ou le facteur de croissance est choisi parmi le groupe composé de l'interleukine-2 (IL-2), l'interleukine-12 (IL-12) et le facteur de stimulation des colonies de granulocytes-macrophages (GM-CSF).

10. Peptide de la télomérase destiné à être utilisé selon l'une quelconque des revendications précédentes, dans lequel la cytokine ou le facteur de croissance est le GM-CSF.

11. Peptide de la télomérase destiné à être utilisé selon l'une quelconque des revendications précédentes, dans lequel le peptide de la télomérase est administré plusieurs fois.

12. Peptide de la télomérase destiné à être utilisé selon l'une quelconque des revendications précédentes, dans lequel le peptide de la télomérase est administré par voie intracutanée avec le GM-CSF.

13. Peptide de la télomérase destiné à être utilisé selon l'une quelconque des revendications précédentes, dans laquelle le cancer est choisi parmi le groupe composé du cancer du sein, du cancer de la prostate, du cancer du pancréas, du cancer colorectal, du cancer du poumon, du mélanome malin, des leucémies, des lymphomes, du cancer des ovaires, du cancer cervical et des carcinomes des voies biliaires.

**Fig. 1**

**Fig. 2**

EP 2 316 476 B1

Fig. 3

65

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- NO 9200032 W
- WO 9214756 A
- EP 10154215 A

### Non-patent literature cited in the description

- **BOON et al.** *Cell,* 1989, vol. 58, 293-303
- **HANS-GEORG RAMMENSEE ; THOMAS FRIEDE ; STEFAN STEVANOVIC.** *Immunogenetics,* 1995, vol. 41, 178-228
- **BARINAGA.** *Science,* 1992, vol. 257, 880-881
- **MALE et al.** Advanced Immunology. J.B. Lippincott Company, 1987
- **M.K. GJERTSEN et al.** *Int. J cancer,* 1997, vol. 72, 784
- **GREIDER ; BLACKBURN.** *Cell,* 1985, vol. 43, 405-413
- **MEYERSON et al.** *Cell,* 1990, vol. 1197, 785-795
- **NAKAMURA et al.** *Science,* 1997, vol. 277, 955-959
- **COLLINS et al.** *Cell,* 1995, vol. 81, 677-686
- **HARRINGTON et al.** *Science,* 1997, vol. 275, 973-977
- **NAKAYAMA et al.** *Cell,* 1997, vol. 88, 875-884
- **HARLEY et al.** *Cold Spring Harbor Symp. Quant. Biol.,* 1994, vol. 59, 307-315
- **KIM et al.** *Science,* 1994, vol. 266, 2011-2015
- **BROCCOLI et al.** *PNAS USA,* 1995, vol. 92, 9082-9086
- **COUNTER et al.** *Blood,* 1995, vol. 85, 2315-2320
- **HIYAMA et al.** *J. Immunol.,* 1995, vol. 155, 3711-3715
- **COUNTER et al.** *PNAS USA,* 1994, vol. 91, 2900-2904
- **SHAY ; BACHETTI.** *Eur. J. Cancer,* 1997, vol. 33, 787-791
- **KLINGELHUTZ et al.** *Nature,* 1996, vol. 380, 79-82
- **SHARMA et al.** *Ann Oncol,* 1997, vol. 8 (11), 1063-1074
- **AXELROD.** *Nature Med,* 1996, vol. 2 (2), 158-159
- **HUMINIECKI.** *Acta Biochim Pol,* 1996, vol. 43 (3), 531-538
- **SORIA ; RIXE.** *Bull Cancer,* 1997, vol. 84 (10), 963-970
- **DAHSE et al.** *Clin Chem,* 1997, vol. 43 (5), 708-714
- **MEYERSON et al.** *Cell,* 1997, vol. 90, 785-795
- **DERES.** *Nature,* 1989, 342
- **MANIATIS et al.** Molecular Cloning: A Laboratory Manual. Cold Spring Harbor Laboratory Press, 1991
- **HARLOW ; LANE.** Antibodies: A Laboratory Manual. Cold Spring Harbor Laboratory Press, 1988